(19)

Europäisches Patentamt
European Patent Office
Office européen des brevets

(11) **EP 4 513 184 A1**

(12) **EUROPEAN PATENT APPLICATION**
published in accordance with Art. 153(4) EPC

(43) Date of publication:
26.02.2025 Bulletin 2025/09

(21) Application number: 23791962.6

(22) Date of filing: 21.04.2023

(51) International Patent Classification (IPC):
$G01N\ 33/483^{(2006.01)}$  $G01N\ 33/543^{(2006.01)}$
$G01N\ 33/545^{(2006.01)}$  $G01N\ 33/552^{(2006.01)}$
$G01N\ 35/00^{(2006.01)}$  $G01N\ 37/00^{(2006.01)}$

(52) Cooperative Patent Classification (CPC):
G01N 33/483; G01N 33/543; G01N 33/545;
G01N 33/552; G01N 35/00; G01N 37/00

(86) International application number:
PCT/JP2023/016016

(87) International publication number:
WO 2023/204315 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.04.2022 JP 2022071196

(71) Applicant: Tauns Laboratories, Inc.
Shizuoka
4102325 (JP)

(72) Inventors:
• TAKEUCHI, Rikiya
Izunokuni-shi, Shizuoka 410-2325 (JP)
• OTA, Toshiya
Izunokuni-shi, Shizuoka 410-2325 (JP)
• OHTA, Hirokazu
Izunokuni-shi, Shizuoka 410-2325 (JP)

• SHIRAKI, Saaya
Izunokuni-shi, Shizuoka 410-2325 (JP)
• MORI, Ayami
Izunokuni-shi, Shizuoka 410-2325 (JP)
• YAMAUCHI, Taisuke
Hamamatsu-shi, Shizuoka 430-0917 (JP)
• YAMAMOTO, Yoshiatsu
Hamamatsu-shi, Shizuoka 430-0917 (JP)
• CHICHIBU, Takeaki
Izunokuni-shi, Shizuoka 410-2325 (JP)
• NAGASHIMA, Shion
Izunokuni-shi, Shizuoka 410-2325 (JP)
• ANDO, Junichi
Izunokuni-shi, Shizuoka 410-2325 (JP)

(74) Representative: Eidelsberg, Olivier Nathan et al
Cabinet Flechner
22, avenue de Friedland
75008 Paris (FR)

(54) **METHOD AND REAGENT FOR DETECTING TARGET SUBSTANCE**

(57)    A technique that can detect a target substance in a sample with higher sensitivity and at lower cost than a conventional technique is provided, which is a target substance detection method including: in a first reaction field, forming a complex by sandwiching a target substance between a first trapping substance immobilized on a solid phase and a second trapping substance labeled with the labeling substance, and separating a moiety including the labeling substance from the complex and moving it through a liquid filled in a tubular channel by a centrifugal force; and detecting the moiety including the labeling substance by light scattered by the moiety including the labeling substance.

Fig. 1

Y SECOND TRAPPING SUBSTANCE    □ TARGET SUBSTANCE    ⚙ FIRST TRAPPING SUBSTANCE PROVIDED WITH LABELING SUBSTANCE

EP 4 513 184 A1

**Description**

Technical Field

The present invention relates to a detection method and a reagent for detecting a target substance in a sample, and in particular, to a detection method and a reagent that can detect the target substance with high sensitivity and at low cost.

Background Art

An immunoassay has heretofore been known which discovers a disease or qualitatively or quantitatively analyzes therapeutic effects or the like by detecting a particular antigen or antibody associated with the disease as a biomarker. In recent years, there has been a growing demand for highly sensitive detection to detect a trace amount of a biomarker for the purpose of detecting a more effective biomarker. An enzyme-linked immunosorbent assay (also referred to as "ELISA" in the present specification), which is one of the immunoassays, is a method of detecting a target antigen, antibody or nucleic acid contained in a sample solution through the use of enzyme reaction while trapping the target with a specific antibody, an antigen that binds to the target antibody, or a complementary nucleic acid, and it widely prevalent because of its advantages such as a cost.

Although ELISA is an assay capable of quantitatively detecting a target substance, most of the currently prevalent ELISA techniques are operated on a 96-well microplate, have a large number of steps, and require a complicated operation. Furthermore, such ELISA undergoes a plurality of reaction steps and therefore requires a great deal of labor and time for obtaining measurement results. Hence, it is desired to shorten an operating time or a reaction time. Moreover, more highly sensitive detection is demanded.

In order to achieve a highly sensitive detection, it is considered that the reactivity between an enzyme and a substrate is improved. For this purpose, for example, a technique has been proposed in which an immune complex (labeled compound), to which a reporter is added, is liberated from a solid phase and is allowed to react in a free liquid medium (Patent Literature 1). In this Patent Literature 1, components of the immune complex are designed such that a biotin or a functionalized azo dye and an avidin are introduced in the immune complex. For example, an antibody bound to a reporter group via streptavidin-biotin binding is prepared as a labeled antibody. When the immune complex is formed on a solid phase, the reporter group is introduced into the immune complex via streptavidin-biotin binding. Then, an excess of streptavidin is added thereto so that streptavidin in the complex is replaced with the added streptavidin to liberate the reporter molecule from the immune complex. Hence, in this technique, the reporter molecule is detected in a free liquid medium and can thereby be associated with an analyte concentration in a specimen.

Further, in order to detect an immune complex at high sensitivity, a microreactor where reaction is performed in a minute container of a μm scale has been developed by microfabrication technology. If a microreactor having a minute space serving as a reaction field is used, it can be expected that a reaction rate is improved. The realization of an experimental operation such as extraction or separation on a microreactor using a minute channel or reaction chamber is often called "lab on a chip". It has been proposed that a biological material is isolated by forming a microreactor in a disc having a flat circular shape and dispensing a sample solution by centrifugal force caused by rotation (Patent Literature 2). It has also been proposed that immunoassay procedures, which have been manually performed in wells, are implemented in a microreactor by forming a plurality of chambers, channels, reservoirs and the like in a disc having a flat circular shape and carrying out the liquid feed of a reagent and waste liquid disposal by rotation of the disc (Patent Literature 3).

A method using a labeling bead in an immunoassay in a microreactor has been proposed for the purpose of detecting a target substance having a low concentration (Patent Literature 4). For example, Patent Literature 4 discloses a disc having a flat circular shape, which is provided with a bead filled portion which is filled with a labeling bead of which surface is modified with an antibody, as well as a detection region. An antigen in a sample solution injected to the disc is trapped by the antibody on the labeling bead, and a labeling bead complex bound by the antigen on the labeling bead is sent to the detection region by the rotation of the disc and trapped by an antibody immobilized on a detection area. It is disclosed that the number of the labeling bead complexes in a state of thus being fixed in the detection area are counted by an optical reading means of an optical disk device, thereby detecting the target substance in the sample.

On the other hand, an analysis method using a disk has been proposed in which antigen-carrying beads, a fluorescent IgG antibody-labeling reagent and a sample are mixed in a reaction field provided in the disk so as to form a complex bound to a target substance, then the beads are introduced from the reaction field into a separation chamber filled with a density gradient medium by a centrifugal force generated by rotation of the disk, and the complexes are detected by irradiation with an electromagnetic irradiation beam. The separation chamber is filled with a density gradient medium, and the introduced beads move to an isopycnic point in the density gradient medium by the centrifugal force, and stop there. Then, the target immune complex can be detected by detecting fluorescence from the isopycnic point. According to this analysis method, excess fluorescent labels and the like, which have not been bound to any immune complex but have an isopycnic point different from that of the beads, can be separated from the target immune complex, and thus the immune complex can be detected without performing any washing operation (Patent Literature 5).

Citation List

Patent Literature

Patent Literature 1 Japanese Patent Laid-Open (kohyo) No. H06-505802
Patent Literature 2 Japanese Patent Laid-Open (kokai) No. 2008-185423
Patent Literature 3 Japanese Patent Laid-Open (kohyo) No. 2002-503331
Patent Literature 4 Japanese Patent Laid-Open (kokai) No. 2012-255772
Patent Literature 5 Japanese Patent Laid-Open (kohyo) No. 2006-505766

Summary of Invention

Technical Problem

However, Patent Literature 1 merely discloses a technique in which a reporter molecule serving as a labeling substance is liberated into a free liquid medium and reacted with a substrate in the liquid. This is insufficient for achieving improvement in detection sensitivity or improvement in reactivity so as to be capable of detecting a low concentration of a target substance.

Patent Literature 2 is silent about immunoassay which is performed with great sensitivity. Patent Literature 3 only discloses one in which the steps of conventional ELISA are implemented in a microreactor. Furthermore, the microreactor is problematic in that signal intensity is decreased with decrease in reaction capacity, though a smaller reaction field can improve a reaction rate or reactivity. Hence, in the case of carrying out measurement of immune complex with high sensitivity by the techniques described in Patent Literatures 2 and 3, a highly sensitive detector or camera is necessary, leading to a problem of high cost.

Patent Literature 4 discloses a technique of carrying out immunoassay using a general-purpose optical disc device by establishing labeling beads to be detected. In general, the transport of granular matter in a microreactor is responsible for clogging. The technique of Patent Literature 4 causes partial accumulation of labeling beads and disadvantageously makes actual handling difficult in some cases because an antibody is immobilized on a solid-phase in a detection region and antigen-carrying labeling beads that flow to this detection region are trapped. Furthermore, the labeling beads trapped in the detection region, when coming close to each other, are disadvantageously responsible for detection errors.

In addition, in the technique described in Patent Literature 4, antigen-antibody reaction is performed between an antibody immobilized on a solid phase and an antigen carried by labeling beads while the labeling beads are travelled. Therefore, the whole detection region serves as a reaction field for the antigen-antibody reaction. Since reactivity is influenced by the frequency of contact between the antigen and the antibody, antigen-antibody reaction efficiency tends to be low in this technique in which the whole area of the detection region serves as a reaction field. Furthermore, the trapping of the labeling beads and the detection thereof (i.e., the detection of the antigen) are performed in the same reaction field. Therefore, detection sensitivity is reduced if reaction efficiency is low. Moreover, the labeling beads are difficult to be trapped by the antibody immobilized on a solid phase, depending on the traveling speed of the antibody. Therefore, unfortunately, detection sensitivity or detection rate becomes poorer.

In addition, the technique described in Patent Literature 5 performs the detection by collecting (unevenly distributing) the beads carrying the complex to an isopycnic point in a density gradient medium. Because of this, more advanced signal detection is required as the accumulation amount decreases, and it is difficult to detect the target substance in the sample with higher sensitivity.

The present invention has been made in view of the above problems, and an object of the present invention is to provide a technique that can detect a target substance in a sample with higher sensitivity and at lower cost than the conventional technique.

Solution to Problem

The present invention according to one aspect provides a target substance detection method, which includes:

forming a complex by sandwiching a target substance between a first trapping substance bound to a labeling substance and a second trapping substance immobilized on a solid phase, and separating a moiety including the labeling substance from the formed complex;

moving the separated moiety including the labeling substance through a liquid filled in a tubular channel by a centrifugal force; and

detecting the moiety including the labeling substance by light scattered by the moiety including the labeling substance from light irradiated into the tubular channel.

According to a preferable embodiment of the present invention, a washing treatment of washing the complex in a state in which the complex is prevented from flowing into the tubular channel is implemented before the moiety including the labeling substance is separated from the complex.

According to a preferable embodiment of the present invention, when the washing treatment is implemented, the tubular channel is filled with a washing liquid used for the washing.

According to a preferable embodiment of the present invention, the washing liquid used for the washing treatment accumulates downstream from the tubular channel.

According to a preferable embodiment of the present invention, the washing treatment is implemented in a state in which the complex is retained in a predetermined chamber, whereby the complex is prevented from flowing into the tubular channel.

According to another preferable embodiment of the present invention, a method of the present invention includes:

arranging a first reservoir that stores the washing liquid, the chamber, and a second reservoir that stores a liquid flowing out from the chamber in a device formed to be rotatable, in this order from a rotation center side of the device toward an outer side; communicating the first reservoir and the chamber with each other through a self-opening and closing flow path, and communicating the chamber and the second reservoir with each other through the tubular channel;

rotating the device, thereby supplying the washing liquid from the first reservoir to the chamber and then discharging the washing liquid to the second reservoir via the tubular channel so as to implement the washing treatment.

According to another preferable embodiment of the present invention, the first reservoir and the chamber are communicated with each other through an openable flow path.

According to another preferable embodiment of the present invention, the washing treatment is performed until the second reservoir and the tubular channel are filled with the washing liquid.

According to another preferable embodiment of the present invention, the method of the present invention includes:

arranging a third reservoir that stores a liquid containing a reagent for separating the moiety including the labeling substance from the complex, in a side closer to the center of rotation than the chamber of the device while the third reservoir and the chamber communicate with each other through a self-opening or closing flow path; and

rotating the device, thereby supplying a liquid containing the reagent to the chamber after the washing treatment, whereby the moiety including the labeling substance is separated and then the separated moiety including the labeling substance is moved into the tubular channel.

According to another preferable embodiment of the present invention, the third reservoir and the chamber are communicated with each other through an openable flow path.

According to another preferable embodiment of the present invention, scattered light is detected that is intermittently generated from the tubular channel within a predetermined time period.

According to another preferable embodiment of the present invention, the labeling substance is a substance having a specific gravity larger than that of the liquid filled in the tubular channel.

According to another preferable embodiment of the present invention, the labeling substance is a fine particle that contains one selected from the group consisting of a metal, a ceramic, glass and a resin, as a main constituent component.

According to another preferable embodiment of the present invention, the labeling substance is a metal colloidal particle.

According to another preferable embodiment of the present invention, the solid phase is at least one granular body selected from the group consisting of glass particles, ceramic particles, magnetic particles and resin particles.

According to another preferable embodiment of the present invention, the solid phase is a structure of an inside of a container that is used for forming the complex.

According to another preferable embodiment of the present invention, the moiety including the labeling substance is separated from the complex, by at least one treatment selected from the group consisting of heat treatment, pH adjustment treatment, denaturation treatment, oxidation treatment, reduction treatment, enzyme treatment and competitive reaction treatment.

According to another preferable embodiment of the present invention, the first trapping substance is at least one selected from the group consisting of an antibody, an antibody fragment, a modified antibody, an antigen, an aptamer and a nucleic acid, each of which specifically binds to the target substance.

According to another preferable embodiment of the present invention, the second trapping substance is at least one selected from the group consisting of an antibody, an antibody fragment, a modified antibody, an antigen, an aptamer and a nucleic acid, each of which specifically binds to the target substance.

According to another aspect of the present invention, a reagent for use in the target substance detection method of the present invention, the reagent having an action of separating the moiety including the labeling substance from the complex.

According to another preferable embodiment of the present invention, the above reagent of the present invention includes at least one selected from the group consisting of a pH adjusting agent, a denaturing agent, a reducing agent, an oxidizing agent, an enzyme and a competing agent.

According to another preferable embodiment of the present invention, the above reagent of the present invention includes the pH adjusting agent.

Advantageous Effects of Invention

According to the target substance detection method of the present invention, the moiety including the labeling substance (also referred to as "labeling substance-containing fragment" in the present specification), which has been separated from the complex, is moved through the liquid filled in the tubular channel by the centrifugal force, and the labeling substance-containing fragment is detected by scattered light that is scattered by the labeling substance-containing fragment which reflects the light irradiated into the tubular channel. In general, as a signal generation source (a substance generating an optical signal, that is, a labeling substance) in a detection target becomes smaller, a higher technique is required for detecting the detection target with the use of a change in absorbance as a signal. However, the method of the present invention acquires scattered light as an optical signal, and accordingly, can sufficiently detect a minute detection target such as the above labeling substance-containing fragment.

Here, when the detection target is detected with the use of scattered light, the scattered light is also generated from a substance other than the detection target, and accordingly, such a problem occurs in many cases that the detection target cannot be specifically detected. However, in the method of the present invention, the labeling substance-containing fragment in the liquid moves at a unique moving speed by the action of the centrifugal force, and accordingly, the labeling substance-containing fragment which is the detection target can be specifically detected. Furthermore, in the method of the present invention, the labeling substance-containing fragment moves through the liquid filled in the tubular channel by the action of the centrifugal force, and accordingly, moves at a slower speed than in the case where the labeling substance-containing fragment is transferred together with the flow of the liquid. Therefore, according to the method of the present invention, even while the labeling substance-containing fragment is moving in the tubular channel by the centrifugal force, it is possible to easily acquire the scattered light emitted from the labeling substance-containing fragment.

Furthermore, according to the method of the present invention, for example, in the case where beads have been used as the solid phase, even if a plurality of target substances have been carried on one bead, counting is not done per unit of bead, but the number of the labeling substance-containing fragments separated from the beads can be sequentially detected (counted); and accordingly, the method exhibits an effect of being capable of detecting the target substance with high sensitivity.

According to the reagent of the present invention, it is possible to satisfactorily separate the moiety including the labeling substance from the complex, in other words, the labeling substance-containing fragment, and accordingly, it is possible to provide a reagent suitable for detection of the target substance.

Brief Description of Drawings

Figure 1 is a diagram illustrating an outline of one embodiment of a detection method of the present invention.
Figure 2a is a schematic plan view showing an example of a disk suitable for implementing a detection method of a first embodiment.
Figure 2b is a partially enlarged view of Figure 2a.
Figure 3 is a schematic plan view showing another example of a disk suitable for implementing the detection method of the first embodiment.
Figure 4 is a schematic view showing one example of a detection device.

Figure 5 is a graph showing a relationship between a pH value and cleavage performance of each reagent in Example 1.

Figure 6 is a graph showing a relationship between a concentration (pH value) of NaOH and cleavage performance in Example 2.

Figure 7 is a graph showing cleavage performance of various denaturing agents in Example 3.

Figure 8 is a graph showing changes in diameters of gold colloidal particles due to sensitizing treatment in Example 4.

Figure 9 is a graph showing a relationship between a particle diameter of metal colloidal particles and an intensity of scattered light in Example 5.

Figure 10 is a graph showing a relationship between a concentration (corresponding to an antigen concentration) of gold colloidal particles (moieties including gold colloidal particles) which were eluted in Example 6 and the intensity of the scattered light.

Figure 11 is a view illustrating selective outflow of a gold colloidal particle (a moiety including a gold colloidal particle) in Example 7.

Figure 12 is a graph showing such a result that the gold colloidal particles (moieties including gold colloidal particles) were measured in Example 7.

Figure 13a is a view showing such a result that the light scattered by the gold colloidal particles (moieties including gold colloidal particles) were detected as a voltage value in Example 8.

Figure 13b is a partially enlarged view of Figure 13a.

Figure 14a is a view showing such a result that the light scattered by gold colloidal particles (moieties including gold colloidal particles) were detected as the voltage value, in the (blank) case where antigens were not used for formation of immune complexes in Example 8.

Figure 14b is a partially enlarged view of Figure 14a.

Figure 15 is a graph showing a relationship between the number of counted spots and the antigen concentration, which was measured in Example 8.

Description of Embodiments

<Detection method of the present invention>

In the detection method of the present invention, a moiety including a labeling substance separated from a complex is moved through a liquid filled in a tubular channel by a centrifugal force, and accordingly, the moiety including the labeling substance is intermittently supplied into the liquid filled in the tubular channel. Then, the irradiation light irradiated into the tubular channel is scattered by the moiety including the labeling substance, and by the thus-generated scattered light, the moiety including the labeling substance is detected. The complex is formed by sandwiching a target substance between a first trapping substance bound to the labeling substance and a second trapping substance immobilized on a solid phase. Because of this, the target substance can be detected through a process of detecting the moiety including the labeling substance separated from the complex.

Note that in the present specification, the case where each of elements is connected by hyphen as in "solid phase-second trapping substance-target substance-first trapping substance-labeling substance" means that the elements are physically or chemically bound to each other. In addition, there is a case where "first trapping substance-labeling substance" is described as "first trapping substance bound to labeling substance" or simply "first trapping substance". "Outer side", "outside direction" and "outward" mean a centrifugal direction. "Main constituent component" is a constituent component having the highest content, and for example, refers to a component having a content exceeding 50% by mass.

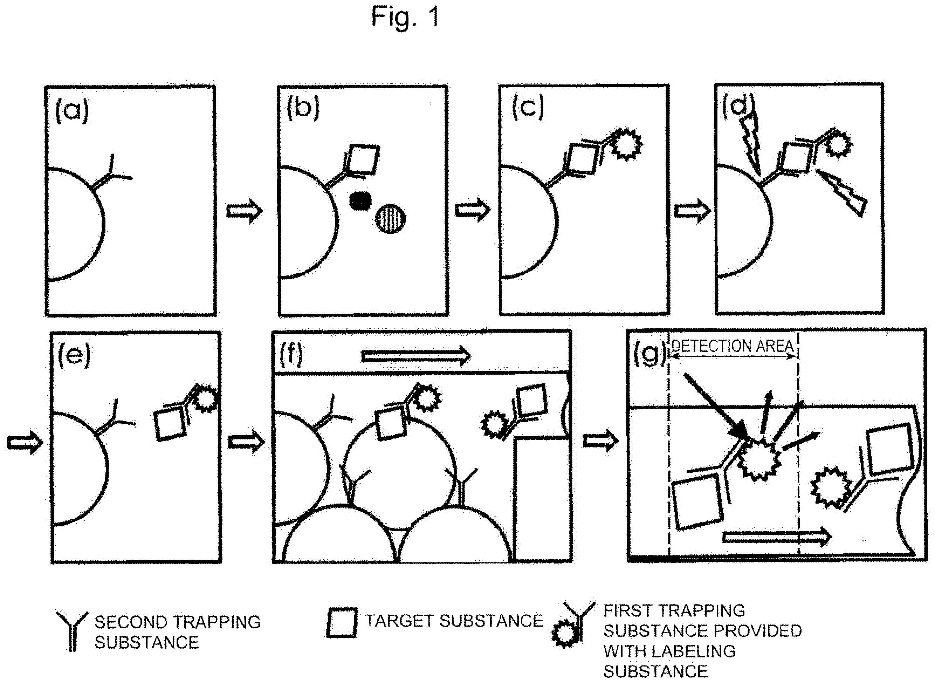

One embodiment of the detection method of the present invention will be described below with reference to Figure 1. Figure 1 is a schematic view showing a concept of one embodiment of the detection method of the present invention. Note that for the sake of simplicity, Figure 1 shows a case where the target substance is an antigen, and the first and second trapping substances are primary antibodies.

In the initial state, a solid phase is prepared on which a second trapping substance for trapping a target substance is fixed, in a reaction field (the first reaction field, in other words, a space where a reaction is implemented) (step 1 in Figure 1(a)). When a sample solution containing the target substance is introduced into the reaction field (Figure 1(b)), the target substance in the sample solution is specifically trapped by a second trapping substance (step 2). Substances other than the trapped target substance are removed from the reaction field by washing treatment as needed, then, the first trapping substance bound to the labeling substance is introduced into the reaction field, the first trapping substance binds to the target substance, and an immune complex is formed in which the target substance is sandwiched between the second trapping substance and the first trapping substance (hereinafter, also simply referred to as "immunocomplex"), on the solid phase (step 3 in Figure 1(c)). Subsequently, it is preferable to perform washing treatment. It is preferable to remove substances that affect a detection result if mixed in the tubular channel, to the outside of the system in advance, by the washing treatment; and examples of such substances include an excess first trapping substance that fails to form the complex and a free labeling substance. In addition, other foreign substances may be removed together by this washing treatment. Thereby, the labeling substance in an amount corresponding to the target substance trapped on the solid phase becomes a state of being carried on the solid phase.

Then, a reagent such as an enzyme, a redox agent, a denaturing agent, an acid or a base, or an external field such as heat, light (electromagnetic waves) or vibration is imparted to the formed complex (Figure 1(d)); and the moiety including the labeling substance is separated from the solid phase (step 4 in Figure 1(e)). Note that the separated portion (moiety including labeling substance) may be only the labeling substance, or may be in a state in which a molecule group is bound to the labeling substance. The molecule group is not limited to a substance in which the original molecule group remains as it is (in other words, a substance which has been generated by cleavage of intermolecular bonds) such as a complex of the second trapping substance-target substance-first trapping substance, a complex of the target substance-first trapping substance, and the first trapping substance, and may also be a substance which has been fragmented by the cleavage of the intramolecular bonds in the original molecule group.

This reaction field is a field in which a reaction that forms the complex by sandwiching the target substance between the first trapping substance bound to the labeling substance and the second trapping substance immobilized on the solid phase, or that separates a moiety including the labeling substance from the formed complex, is implemented; and means a partitioned space. Examples of the reaction field include a reaction vessel and a reaction compartment (chamber). Meanwhile, in this method, steps 1 to 4 shown in the above Figures 1(a) to 1(e) may be implemented in the respective separated reaction fields, , or a plurality of successive steps may be implemented in the same reaction field, as long as the moiety including the labeling substance can be moved in the tubular channel by the centrifugal force. It is also acceptable, for example, to complete steps 1 to 3 with the use of a single container, and complete step 4 in another single container.

Note that in the present embodiment, it is acceptable to use a reaction chamber provided in a device configured to be rotatable, as the reaction field. The stage at which the use of the reaction chamber begins can be arbitrarily selected from steps 1 to 4, including the washing treatments to be appropriately implemented. For example, it is acceptable to load the solid phase on which the second trapping substance is fixed into a reaction chamber of the above device, and to implement all of steps 1 to 4 with the use of the reaction chamber, or alternatively to implement steps 1 to 3 in a container outside the above device, and to implement step 4 and the subsequent steps in the reaction chamber of the above device. In the latter case, regardless of whether or not the product of step 4 is subjected to solid-liquid separation, a solution which contains the moiety including the labeling substance is introduced into the reaction chamber, but in such a case, the reaction chamber shall be used simply as a front chamber for supplying the solution to a detection field, in other words, the tubular channel having a minute diameter.

After step 4, the moiety including the labeling substance, which is contained in the solution, is moved into the detection field by the centrifugal force, in other words, into the liquid filled in the tubular channel having the minute diameter (Figure 1(f)). In an example illustrated in Figure 1(f), the tubular channel having the minute diameter is continuously provided from the reaction chamber toward the outer side direction (right side in the figure) than the rotation center. Accordingly, if the moiety including the labeling substance is stored in the reaction chamber of the device in a state of being contained in the solution, the moiety including the labeling substance can enter the liquid filled in the tubular channel having the minute diameter and move further outward through the liquid by the centrifugal force generated when the device is rotated. In addition, in the illustrated example, a pore diameter of the tubular channel having the minute diameter is set to such a size as to allow the moiety including the labeling substance to pass therethrough but not to allow the solid phase to pass therethrough, and accordingly, the tubular channel can allow the moiety including the labeling substance to progress thereinto while preventing the solid phase from flowing thereinto. Here, the moiety including the labeling substance moves through the liquid filled in the tubular channel by the centrifugal force. The movement is due to so-called centrifugal sedimentation. In other words, the moving speed of the moiety including the labeling substance is relatively larger than a moving speed of the liquid filled in the tubular channel. In the present invention, the liquid filled in the tubular channel is hardly moved by the centrifugal force, and usually, the moving speed is approximately zero, but in principle, the liquid is allowed to slightly move as long as the movement does not substantially affect the movement of the moiety including the target substance.

The other end of the tubular channel is connected to a second reservoir (not illustrated in Figure 1) provided in the above device. The tubular channel and the second reservoir may be always communicated with each other, or may be self-opening and closing by a valve. The second reservoir may be provided downstream from the chamber in the device, and is preferably provided at a position outward from the chamber, for example. Before the moiety including the labeling substance is moved to the tubular channel, the tubular channel is set to a state of being filled with a liquid. In the case where the second reservoir is provided at a position outward from the chamber, the tubular channel and the second reservoir always communicate with each other, and both of the tubular channel and the second reservoir are filled with the liquid. The timing when the tubular channel is filled with the liquid differs according to the timing when the reaction chamber is used. For example, in the case where the reaction chamber is used before the previously described step 3, the tubular channel is filled with the liquid after the washing treatment after the step 3, and before the step 4. In addition, in the case where the reaction chamber is used in step 4 or later, the tubular channel is preferably filled with the liquid before the solution which contains the moiety including the labeling substance is introduced into the reaction chamber. Note that the reaction chamber may be configured to be provided with a valve such that after the tubular channel has been completely filled with the liquid, the valve is opened to introduce the moiety including the labeling substance into the liquid filled in the tubular channel. In such a case, the timing when the solution which contains the moiety including the labeling substance is introduced into the reaction chamber may be before or after the time when the tubular channel has been completely filled with the liquid.

The detection field (which may be hereinafter referred to as "second reaction field" as appropriate) is a field for detecting scattered light emitted from the tubular channel, and is provided with a light source and a light receiving element; and is a field where an optical change is caused by generating scattered light based on irradiation light, . Because at least a part of the tubular channel which is irradiated with the irradiation light is formed from a translucent material, the irradiation light which has been emitted from the light source toward the tubular channel passes through a wall of the tubular channel, and is scattered by the labeling substance moving in the tubular channel; and scattered light is generated. The scattered light is detected by a light receiving element, and thereby the presence or absence of the labeling substance can be detected (Figure 1(g)).

A moving speed of a particle which has been placed in a centrifugal field in a liquid is described by Stokes' law. Specifically, when a particle having a mass m is rotated at a rotation radius $r_1$ and a rotation angular speed $\omega$, the particle receives a centrifugal force f as in the following Expression (1).

$$f = mr_1\omega^2 \quad \ldots\ldots (1)$$

m: mass of particle (g)
$r_1$: rotation radius (cm)
$\omega$: rotation angular speed (rad/s)

In addition, when a radius of the particle is represented by $r_2$, a particle density is represented by $\rho_p$, and a liquid density is represented by $\rho_l$, a centrifugal force Fc which acts on the particle is expressed by the following Expression (2).

$$F_c = ma = \frac{4\pi r_2^{\,3}}{3}(\rho_p - \rho_l)r_1\omega^2 \cdot \cdot \cdot \cdot \cdot \cdot (2)$$

According to Stoke's law of resistance, the resistance Fr that the sphere having a radius of $r_2$ receives, which moves at a speed vs in a liquid having a viscosity $\eta$, is expressed by the following Expression (3).

$$Fr = 6\pi\eta r_2 v_s \quad \ldots\ldots (3)$$

Fc and Fr are balanced at a terminal speed of the particle, and accordingly, the following Expression (4) is obtained; and when the radius $r_2$ of the particle in the Expression (4) is replaced with a diameter $D_p$, the moving speed $v_s$ of the particle is expressed by the following Expression (5).

$$6\pi\eta r_2 v_s = \frac{4\pi r_2^{\,3}}{3}(\rho_p - \rho_l)r_1\omega^2 \quad \cdot \cdot \cdot \cdot \cdot \cdot (4)$$

$$v_s = \frac{D_p^2(\rho_p - \rho_l)r_1\omega^2}{18\eta} \cdot \cdot \cdot \cdot \cdot \cdot (5)$$

Specifically, when the centrifugal force acts, the moving speed ($v_s$: cm/sec) of a particle is determined in relation to the diameter ($D_p$: cm) of the particle, the density ($\rho_p$: $g \cdot cm^{-3}$) of the particle, the density ($\rho_1$: $g \cdot cm^{-3}$) of the liquid, a viscosity ($\eta$: poise; $g \cdot cm^{-1} \cdot s^{-1}$) of the solution, and the like. In other words, in the same liquid, a particle larger in particle density or particle diameter results in moving faster in the liquid. As for a particle of which density or particle diameter is known in advance, it is possible to estimate the moving speed and the moving time period to a predetermined position, on the basis of Expression (5).

It is also possible to grasp the particle diameter and density of the labeling substance, and further the viscosity of the solution, in advance. In addition, in the case where the labeling substance in the moiety including the labeling substance is sufficiently larger or heavier than a portion other than itself, it is possible to approximate the moiety including the labeling substance by the labeling substance. Thereby, it becomes possible to derive the moving speed of the moiety including the labeling substance and the arrival time at the predetermined position (detection area) of the tubular channel, theoretically on the basis of Expression (5) or by experiment. Note that when the condition deviates from the precondition of Expression (5), it is also acceptable to calculate the moving speed and the like after correcting Expression (5), because Expression (5) is a theoretical expression.

As described above, it is possible to estimate a time period for the moiety including the labeling substance to pass through the detection area, on the basis of the derived moving speed. In addition, the moiety including the labeling substance passes through the detection area during a predetermined time period from the start of centrifugation. Therefore, it can be determined that the scattered light which is detected in the detection area during the predetermined time period is scattered light which is caused by the detection target of interest.

Furthermore, the detection target is a substance which moves in the tubular channel, and accordingly, the scattered light becomes intermittent signals. Because of this, it becomes possible to identify the scattered light which is continuously generated as a background noise due to some factors, and to specifically detect the scattered light of the detection target.

In this way, in the detection method of the present invention, it is possible to appropriately detect a minute detection target. The labeling substance (moiety including labeling substance), which has been supplied to the tubular channel, is derived from the substance that has formed a complex with the target substance in the reaction field, and accordingly, it becomes possible to detect the presence of the target substance, by detecting the labeling substance.

In addition, as for the scattered light which is intermittently generated in such a predetermined time period, it is possible to count the number of times of the generation. It is possible on the basis of this count to quantitatively evaluate the number of moieties including the labeling substance which have been supplied to the tubular channel, that is, the number of the target substances. In particular, in the case of microanalysis in which the number of target substances in a sample is small, the detection method is quantitatively excellent.

Furthermore, the detection method of the present invention includes separating the moiety including the labeling substance from the solid phase, and introducing the separated moiety including the labeling substance into the detection field; and accordingly can significantly reduce the size of the substance which passes through the flow path, as compared with the case where the immunoassay is performed by counting the beads on which the immunocomplex is formed with the use of a disk-like microreactor that is conventionally proposed, and can smoothly and easily introduce the substance of the detection target into the detection field.

<Target substance>

The target substance to be analyzed in the measurement method of the present invention is a substance that binds to a specific biomolecule, and may also be a substance that binds to a specific biomolecule by a biochemical reaction. The target substance is, for example, a substance which can be detected immunologically or genetically, and is not particularly limited. Specific examples of the target substance include: pathogens such as bacteria and viruses; proteins, peptides; nucleic acids such as DNA, RNA and aptamers; exosomes; and various chemical compounds such as sugar chains. Furthermore, examples of the target substance include physiologically active substances such as hormones, agonists and antagonists thereof, alkaloids and the like.

<First trapping substance>

The first trapping substance of the present invention binds to the target substance which has been trapped by the second trapping substance, and sandwiches the target substance between itself and the second trapping substance immobilized on the solid phase so as to form a complex fixed to the solid phase, in other words, a complex in which the solid phase - the second trapping substance - the target substance - the first trapping substance (first trapping substance bound to labeling substance) are bound in this order. When the target substance is an antigen, an antibody is used which specifically reacts with the target substance, as the first trapping substance. On the other hand, when the target substance is an antibody, as the first trapping substance: an antigen may be used to which the antibody specifically binds; or alternatively it may be another antibody that binds to the antibody and is different from the second trapping substance; and furthermore, an antibody which has been produced by a host different from a host which produces the antibody may be used. Because of this, when the target substance is an antibody, a complex of solid phase-antigen (second trapping substance)-target substance-antigen (first trapping substance) or antibody (first trapping substance), or a complex of solid phase-antibody (second trapping substance)-target substance-antigen (first trapping substance) or antibody (first trapping substance), is formed.

Furthermore, as the first trapping substance of the present invention other than the above described antigen and antibody, it is acceptable to adopt a nucleic acid, an aptamer, any one of biotin-avidin, any one of ligand-receptor, or the like, according to the target substance. For example, when the target substance is DNA derived from a bacterium, a nucleic acid molecule that has a sequence complementary to a partial region of the DNA can be used as the first trapping substance. In addition, in the case where the target substance is a sequence-specific DNA-binding protein such as a transcription factor, it is possible to use a DNA having a specific sequence as the first trapping substance.

An antibody to be used as the first trapping substance may be a polyclonal antibody or a monoclonal antibody, but is preferably the monoclonal antibody, from the viewpoint of reaction specificity.

The antibody to be used as the first trapping substance includes an antibody, and also an antibody fragment and a modified antibody which have substantially the same reactivity as the antibody. Examples of the antibody fragment include a Fab fragment, a F(ab')2 fragment, a Fab' fragment, and a scFv fragment.

<Second trapping substance>

The second trapping substance of the present invention has a function of trapping a target substance on a solid phase, and is used by being fixed to the solid phase; and for example, in the case where the target substance is an antigen, an antibody that specifically reacts with the target substance is used, and in the case where the target substance is an antibody, an antigen or antibody which the antibody specifically reacts with is used.

Furthermore, as the second trapping substance of the present invention, in addition to the above described antigen and antibody, it is also acceptable to use a nucleic acid, an aptamer, a ligand (a substrate, an inhibitor or an antagonist of a target substance) or a receptor, according to the target substance. For example, in the case where the target substance is a DNA derived from a bacterium, it is possible to use a nucleic acid molecule which has a sequence complementary to a partial region of the DNA, as the second trapping substance. In addition, in the case where the target substance is a sequence-specific DNA-binding protein such as a transcription factor, it is possible to use a DNA having a specific sequence, as the second trapping substance.

An antibody to be used as the second trapping substance may be a polyclonal antibody or a monoclonal antibody, but is preferably a monoclonal antibody, from the viewpoint of reaction specificity.

The antibody to be used as the second trapping substance includes an antibody, and also an antibody fragment and a modified antibody which have substantially the same reactivity as the antibody. Examples of the antibody fragment include a Fab fragment, a F(ab')2 fragment, a Fab' fragment, and a scFv fragment.

Here, when a size of the solid phase is compared with a size of the second trapping substance, the solid phase is much larger. Because of this, a plurality of second trapping substances tend to be easily solid-phased on the solid phase. In a conventional method of detecting a target substance by causing a solid phase such as a bead to trap the target substance, and counting the beads that have trapped the target substance, if a plurality of target substances are trapped on one bead, the detection result may be greatly affected. However, in the present embodiment, the detection target is the moiety including the labeling substance, which has been separated from the complex and no longer constrained to the bead, and accordingly, it is possible to realize highly sensitive detection.

Note that in the case where a steric hindrance of the labeling substance gives a great influence on the access of the first trapping substance to the target substance which has been trapped on the solid phase, it is acceptable to appropriately adjust a concentration or the like of the second trapping substance when the second trapping substance is bound to the solid phase so that the distribution of the second trapping substance on the solid phase is sparse, in other words, at low density.

<Labeling substance>

The labeling substance is not particularly limited as long as the labeling substance labels the first trapping substance and generates scattered light. Examples thereof include fine particles that contain an inorganic substance such as glass, ceramics or metal, and a fluorescent substance or a resin, as a main constituent component. Examples of the resin particles include latex including synthetic latex such as polystyrene latex and natural rubber latex; and particles such as of synthetic resins represented by polystyrene and the like. In addition, the labeling substance may be composed of a single material or a plurality of materials. Furthermore, it is also acceptable that different types of inorganic-organic materials are combined. It is acceptable for the combination of different types of materials to be, for example, composite particles in which metal particles are carried on resin particles or ceramic particles, or to be composite particles in which resin particles are coated with metal. In addition, it is acceptable that a fluorescent substance is carried on a resin particle, but the combination is not limited thereto.

It is preferable that the particle diameters of these labeling substances be 80 nm to 3 $\mu$m, from the viewpoint of the intensity of the scattered light and the mobility in the device. Note that the intensity of the scattered light, the moving speed in the centrifugal field, the reactivity with the first trapping substance and the like vary depending on the type and size of the labeling substance, and accordingly, a labeling substance having an appropriate particle diameter is selected in consideration of these factors.

Here, in consideration of contribution to the moving speed by the centrifugal force, it is preferable for the labeling substance to be a substance having a specific gravity larger than that of the liquid filled in the tubular channel. Inorganic particles having a higher density than organic particles and resin particles having a relatively large particle diameter are suitable as the labeling substance. This is because it is possible to cause the moving speed of the moiety including the labeling substance and the moving speed of the organic substances which are the main foreign substances in the sample to largely differ from each other, in the detection field. Among these, metal particles and metal colloidal particles which can be expected to provide a satisfactory intensity of the scattered light are particularly suitable as the labeling substance.

As the metal colloidal particles which can be used as the labeling substance, various metal colloidal particles can be appropriately selected and used; and examples thereof include particles of metal colloid such as gold colloid, silver colloid, and platinum colloid, and composite metal colloid thereof. Examples of the composite metal colloid include platinum-carried gold colloid and platinum-carried palladium colloid.

In the present invention, preferable examples of the metal colloidal particles include gold colloidal particles, composite metal colloidal particles thereof, or silver colloidal particles; and gold colloidal particles are more preferable.

The particle diameter of the metal colloidal particle is usually 1 nm to 500 nm. The particle diameter of the metal colloidal particle suitable for detection varies depending on sensitivity, accuracy, measurement conditions and the like of an optical detection system to be selected. For example, in the case where such a simple detection system is used as to detect the scattered light by a combination of a laser light source and a photodiode, the particle diameter of the metal colloidal particle may be 80 nm or larger, is more preferably 150 nm or larger, and when being 200 nm or larger, can be further easily detected. The particle diameter is preferably 80 nm to 500 nm, is more preferably 100 nm to 500 nm, is further preferably 120 nm to 300 nm, and is particularly preferably 180 nm to 250 nm.

Note that in the case where the first trapping substance is an antibody, the cases are included in which the antibody that is the first trapping substance is labeled, and in which an antibody (for example, secondary antibody) that recognizes the antibody is labeled.

The method for binding the labeling substance to the first trapping substance is not limited, and for example, such binding methods can be used as binding by physical adsorption, chemical bond, binding which utilizes affinity, and combinations thereof. The labeling substance and the first trapping substance can be bound by known methods.

When sizes are compared between the labeling substance and the first trapping substance, the labeling substance is relatively larger. Accordingly, the case can occur in which a plurality of first trapping substances bind to the labeling substance. When a large number of first trapping substances bind to the labeling substance, the reactivity between the first trapping substances and the target substance is enhanced, but as the concentration of the target substance increases, such a state tends to easily occur that a plurality of target substances correspond to one labeling substance. In this case, the concentration of the target substance is evaluated to be a concentration lower than the actual concentration. Because of this, in order to broaden a concentration range in which quantitative detection is possible, to a higher concentration side, it is acceptable to implement a process for optimizing a ratio between the labeling substance and the first trapping substance. Examples of such a process include a process of adjusting a charging ratio of the first trapping substance bound to the labeling substance to the target substance, in a reaction of causing the first trapping substance bound to the labeling substance to bind to the target substance.

In addition, for the introduction of a sample (moiety including labeling substance) into the detection field, a tubular channel which is a hollow conduit is used, and a tubular channel having a minute diameter is preferably used. The tubular channel having a minute diameter may be, for example, a micro flow path. When the labeling substance is an insoluble substance such as a metal colloidal particle, the particle diameter of the labeling substance bound to the first trapping substance is preferably as small as possible, in order to avoid clogging in the flow path. On the other hand, in view of a point that the intensity of the scattered light decreases as the particle diameter decreases, the labeling substance is preferably selected as appropriate from a particle having a particle diameter in a range of 80 nm to 3 μm, as described above.

Here, in the case where the labeling substance is a metal colloidal particle, it is acceptable to perform, for example, a sensitization reaction of growing the colloidal particle and increasing the particle diameter. According to this reaction, when the first trapping substance and the metal colloidal particle of the labeling substance are bound, a smaller metal colloidal particle is used, and accordingly, the number of first trapping substances which bind to one metal colloidal particle can be controlled. In addition, when the particle diameter of the metal colloidal particle has been increased by the sensitization reaction, before the labeling substance is introduced into the detection field, the intensity of the scattered light can be increased. In the present invention, it is sufficient that the labeling substance is introduced into the detection field, and it is not necessary that the target substance and/or the first trapping substance that binds to the target substance be introduced into the detection field. Because of this, even if the target substance or the first trapping substance bound to the labeling substance is decomposed or denatured by the sensitization reaction, there is no problem.

Specifically, in the case where the labeling substance is a metal colloidal particle, it is possible to reduce metal ions in a metal salt solution that contains gold ions or silver ions, which are separately added, and accumulate the metal ions, on the surface of the metal colloid of the labeling substance, for example, by utilizing the catalytic activity of the metal colloid or using a reducing agent in combination; and can increase the particle diameter of the metal colloid.

As the metal colloidal particle to be subjected to the sensitization reaction, a metal colloidal particle is preferable in which a metal species that is a core is high in stability against oxidation and reduction. Note that the metal colloidal particle which is the core may be formed from a single metal or a composite metal. Examples of such a metal colloidal particle include a gold colloidal particle, a platinum colloidal particle, and a palladium colloidal particle. In addition, examples of a composite metal colloidal particle include a platinum-gold colloidal particle in which a platinum colloidal particle is carried on a gold colloidal particle.

In such a sensitization reaction of the metal colloidal particle, examples of the metal salt which is a supply source of metal ions include salts of metals belonging to the fourth, fifth, and sixth periods of the periodic table; but it is preferable to use a salt of gold, platinum, silver, palladium, nickel, cobalt, copper or the like. The metal to be precipitated may be the same metal as the metal colloidal particle or a different metal; and is not limited as long as the reduction reaction can be implemented under easy conditions.

For example, in case of a palladium-platinum colloidal particle, the colloidal particle can grow by use of a solution containing any metal ion of a nickel ion, a cobalt ion, and a copper ion, as a sensitizing agent solution.

In addition, for example, in case of a gold colloidal particle, the colloidal particle can grow by allowing gold colloid to accumulate on the surface of the gold colloidal particles serving as a labeling substance by using a sensitizing agent solution containing chloroauric acid as a supply source of gold ions. In addition, the gold colloidal particle can grow as a composite metal colloidal particle by allowing a silver colloid to accumulate on the surface of the gold colloidal particle by use of a solution of a sensitizing agent which contains silver nitrate. The platinum-gold colloidal particle can grow utilizing the catalytic activity of platinum with use of a sensitizing agent solution that contains silver nitrate, a copper salt such as copper sulfate, or the like.

The sensitizing agent solution contains various metal salts which become a supply source of the above metal ion as an essential component, and can further contain a complexing agent, a pH adjusting agent, a buffering agent, a stabilization agent and the like, as needed. As such a sensitizing agent solution, it is also possible to use a conventionally known electroless plating solution.

Examples of the complexing agent include ammonia, a citrate, a tartrate and a lactate. Examples of the pH adjusting agent include an acetate, a propionate, and an ammonium salt. Examples of the stabilization agent include various types of surfactants.

In order to accelerate the reduction reaction of the metal ion, it is acceptable to use a solution containing a reducing agent. Examples of the reducing agent include citric acid, ascorbic acid, sodium hypophosphite, hydrazine hydrate, hydrazine sulfate, sodium borohydride, dimethylamine borane, formaldehyde, Rochelle salt, glucose, and ethylene glycol; but are not limited thereto. The reducing agent to be used is appropriately selected from substances suitable for the metal ion to be reduced.

Note that it is acceptable to perform the sensitization reaction with a sensitizing agent solution which contains both a metal ion component and a reducing agent, and in this case, it is also acceptable to perform the sensitization reaction with a mixture of a metal salt solution containing a metal ion component and a reducing agent solution containing a reducing agent, which have been separately prepared and mixed immediately before the sensitization reaction. Preferably, such a method as to separately prepare a metal salt solution and a reducing agent solution, and to add each of the solutions to the reaction system, is preferable. Note that it is also acceptable to implement the sensitization reaction without adding the reducing agent solution, depending on the catalytic activity of the metal colloidal particle.

It is preferable that this sensitization reaction be implemented after the complex has been formed and before the moiety including the labeling substance is separated.

<Method of cutting out moiety including labeling substance>

The detection method of the present invention includes: forming a complex of a second trapping substance-target substance-first trapping substance (first trapping substance bound to labeling substance) on the solid phase, in the reaction field, and then cutting out (separating) the moiety including the labeling substance from the complex, and releasing the moiety from the solid phase into a liquid. The cutting-out and separation are performed in a state in which the complex is impregnated in the liquid. When the tubular channel is filled with the liquid, in addition, the liquid is stored also in the reaction field so that the solid phase is immersed in the liquid, the tubular channel and the reaction field communicate with each other through the liquid. The moiety including the labeling substance in the liquid of the reaction field, which has been separated from the solid phase by the cutting-out, is intermittently supplied into the liquid with which the tubular channel is filled, by the centrifugal force. As described above, the separated moiety only has to contain the labeling substance, and does not need to be in a state of the first trapping substance containing the labeling substance as it is. In other words, any bond in the complex may be cleaved, as long as the labeling substance or the moiety including the labeling substance is separated.

Note that it is preferable that the moiety including the labeling substance separated from the complex does not contain the solid phase (part of solid phase), from the viewpoint of transportability and the like. Accordingly, it is preferable to release the moiety including the labeling substance, by separation of the complex between the solid phase and the second trapping substance, in a middle of the second trapping substance, between the second trapping substance and the target substance, in a middle of the target substance, between the target substance and the first trapping substance, in a middle of the first trapping substance or between the first trapping substance and the labeling substance.

As described above, the method for binding the labeling substance to the first trapping substance is not limited, and for example, such binding methods can be used as binding by physical adsorption, chemical binding, binding which utilizes affinity, and combinations thereof. The labeling substance and the first trapping substance can be bound by known methods.

Examples of the means for cleaving (separating) the moiety including the labeling substance include a method of cleaving by applying physical energy such as heat, light or vibration from an external field, and a method of adding a cleaving reagent constituted by an oxidizing agent, a reducing agent or the like. The cleaving reagent may be a reagent which cleaves a bond between the solid phase and the second trapping substance, or may be a reagent which cleaves another bond in the complex. Such a cleaving reagent is not particularly limited as long as the reagent can separate the moiety including the label from the complex which has been formed on the solid phase and does not remarkably impair the properties of the labeling substance, and is preferably a reagent which cleaves the bond between the target substance and the second trapping substance or the first trapping substance, or between the first trapping substance and the labeling substance.

Examples of the cleaving reagent for cleaving the bond include a pH adjusting agent, a denaturing agent, a reducing agent, an enzyme, a competing agent, and an oxidizing agent; preferable examples include the pH adjusting agent, the denaturing agent, the reducing agent, and the enzyme; and more preferable examples include the pH adjusting agent and the denaturing agent.

The pH adjusting agent is a chemical compound or composition that contains an acid, a base or a salt thereof and changes the pH. The acid may be any of an inorganic acid and an organic acid, and the base may be any of an inorganic base or an organic base. Examples of such acids and bases include Bronsted acids, bases and salts thereof, including, for example, hydrochloric acid, boric acid, carbonic acid, nitric acid, acetic acid, boronic acid, formic acid, citric acid, phosphoric acid, oxalic acid, lactic acid, malic acid, salicylic acid, glycine hydrochloride, glycine sodium hydroxide, sodium hydroxide, amines such as triethylamine, dimethylamine and methylamine, ammonia, and sodium hydrogen carbonate; but are not limited thereto. Among them, strong acids and strong bases are preferable; and specific examples thereof include hydrochloric acid, nitric acid, formic acid, phosphoric acid, oxalic acid, glycine hydrochloride, glycine sodium hydroxide, sodium hydroxide, and amines such as triethylamine, dimethylamine and methylamine.

The denaturing agent is a reagent that changes a higher-order structure of a protein or a nucleic acid, and examples thereof include: protein denaturing agents such as urea and guanidine salts; and surfactants such as sodium dodecyl sulfate. In addition, examples of other denaturing agents include Lewis acids which are not included in the above pH adjusting agents, alcohols, esters, and derivatives thereof; but are not limited thereto.

Examples of the reducing agents include 2-mercaptoethanol and dithiothreitol which cleave disulfide bonds.

The enzyme is a reagent that hydrolyzes a part of the constituent elements of the complex, and examples thereof include pepsin, papain, ficin, nuclease, and enzymes that specifically act on a specific bond of the constituent elements.

Examples of the competing agents include an agonist, an antagonist, and a competing antibody that compete against the binding between the target substance and the first or second trapping substance.

In addition, it is also acceptable to use peroxides including hydrogen peroxide and other oxidizing agents as the cleaving reagent, as long as the reagent does not affect the target substance.

The type and concentration of the cleaving reagent are appropriately selected according to the structure and binding force of the target complex, and are adjusted in such a range that the function and structure of the labeling substance are not remarkably impaired.

Here, it is also acceptable to introduce a linker into a predetermined part of the complex in advance so that an arbitrary part of the complex is specifically cleaved. For example, when the solid phase and the second trapping substance, or the labeling substance and the first trapping substance are bound together, it is possible to use the linker for the purpose of linking these two substances. If the linker has a predetermined functional group or reactive group which can form a chemical bond at each terminus thereof, it is possible to link the solid phase with the second trapping substance, or the labeling substance with the first trapping substance, via the functional groups at both termini of the linker. It is possible to use this linker for the purpose of linking the solid phase with the second trapping substance, or the labeling substance with the first trapping substance; but the purpose is not limited thereto. For example, it is also acceptable to introduce the linker into the inside of the first trapping substance or the second trapping substance. Examples of the functional group or reactive group at both termini of the linker include groups which form a covalent bond such as an amide bond, a disulfide bond and an ester bond. In this case, when a terminal bonding site of the linker is subjected to hydrolysis or treatment with a reducing agent, the bond at the terminal portion of the linker is decomposed, and thereby the linker can cleave the complex.

In addition, a binding mechanism between the linker and the target substance is not limited to the above covalent bond, and may be, for example, binding such as physical adsorption, ionic bonding, binding which utilizes intermolecular affinity, and a combination thereof.

Furthermore, a molecular structure constituting the linker is not limited, and a protein, a peptide, a sugar chain, a nucleic acid, or a synthetic polymer such as polyethylene glycol can be used. In addition, it is acceptable to use biotin-avidin which forms an affinity bond, or the like.

Such a linker part can be cleaved by physical energy from an external field or a reagent. Usable reagents include an enzyme such as an endopeptidase, a glycolytic enzyme and an endonuclease, a reducing agent, an acid, a base, a surfactant, and a competing agent, according to the type of the linker.

Specifically, it is possible to cleave the linker part by causing, for example, a reducing agent having a thiol group in the molecule such as 2-mercaptoethanol or dithiothreitol to act on the disulfide bond. In addition, in the case of biotin-avidin binding, it is possible to cleave the bond by changing a pH of the reaction system by addition of a pH adjusting agent, or by using an elution buffer. Furthermore, in the case where the linker molecule is composed of a sugar chain, it is possible to cleave a specific bond by causing an enzyme specific to a bond between constituting monosaccharides to act thereon.

Furthermore, in the case where a linker having a histidine tag is used, it is possible to immobilize the second trapping substance on the solid phase via the histidine tag, for example, by binding a linker molecule having the histidine tag at the terminal to the second trapping substance, and fixing nickel on the surface of the solid phase with a chelating agent. When histidine or imidazole which is a competing agent for the histidine tag is added as a cleaving reagent, it is possible to dissociate the histidine tag from nickel, and accordingly it is possible to separate the complex containing the labeling substance from the solid phase.

The cleaving reagent for separating the moiety including the labeling substance from the complex may be removed or may be inactivated after the cleaving reaction. This corresponds to, for example, a treatment in which when the reaction field is inclined from neutral to acid or alkali due to the addition of a pH adjusting agent, a pH adjusting agent is further added to return to neutrality.

The reagent of the present invention is configured to include the above-described cleaving reagent. Note that the reagent of the present invention may be configured to be in a form of a reagent kit. The reagent kit may include, for example, any one or more of a washing liquid, a dispersion liquid in which the first trapping substance bound to the labeling substance is dispersed, and a dispersion liquid of a solid phase on which the second trapping substance is carried, in addition to the reagent (cleaving reagent) of the present invention.

<Solid phase>

The solid phase is an insoluble solid to which the second trapping substance binds. The solid phase is processed into such a form, shape and size as to be capable of being placed in the reaction field.

The form or shape of the solid phase is not particularly limited, but may be any of a structure such as an inner wall of the reaction vessel serving as the reaction field or a projection or structural object which is formed on the inner side of the reaction vessel so as to be integrated with the reaction vessel, and a structure which is formed separately from the reaction vessel.

Examples of the solid phase that is formed separately from the reaction vessel include a plate-like body, a granular body, a fibrous body, a woven fabric, a nonwoven fabric, a film, and a sheet, any of which can be accommodated in the reaction vessel. The plate-like body, the granular body, and the fibrous body may be any of hollow, solid and porous bodies. The shape of the granular body may be any of a sphere, an annular body, a flat body, a columnar body, an irregular shaped body, and the like. The granular body may be at least one selected from, for example, magnetic particles, glass particles, ceramic particles, and resin particles which are formed from a polymer such as latex or polystyrene. It is preferable for the solid phase to be glass particles, ceramic particles, magnetic particles or resin particles.

As such a solid phase, the granular body, the fibrous body, the woven fabric or the nonwoven fabric is preferably used, more preferably the granular body or the fibrous body is used, and further preferably the granular body is used.

Note that it is possible to utilize binding modes, for example, such as physical adsorption, covalent bonding, ionic bonding, binding which utilizes affinity, and combinations thereof, for the binding between the solid phase and the second trapping substance. Preferably, the second trapping substance is immobilized on the solid phase by bonding with a covalent bond or an ionic bond, which is stronger than a binding force due to affinity or adsorption force. In addition, the second trapping substance is solid-phased on the solid phase, in an amount necessary for trapping the maximum detectable amount of the target substance, in a set detection range (concentration range of target substance set as detectable).

The granular body having a larger specific surface area is preferable in view of trapping the target substance, but in the case where the granular body is introduced into the reaction chamber communicating with the tubular channel, the granular body that has a size which does not flow into the tubular channel from the inlet of the tubular channel is selected. For example, when the smallest dimension of the length, width and thickness of the granular body is about 1.2 to 5 times the inlet diameter, the granular body is sufficiently prevented from flowing into the tubular channel. Note that in the case where the inlet shape of the tubular channel is anisotropic, the inlet diameter is defined to be the dimension in the minor axis direction. As the granular body, various beads having a diameter of about 1 $\mu$m to 100 $\mu$m are available as commercial products. Accordingly, it is possible to appropriately select a suitable one according to a size of the inlet. It is preferable for the granular body to be used to be a resin bead, is further preferable to be a resin bead having a particle diameter of 10 $\mu$m to 100 $\mu$m, is more preferable to be a resin bead having a particle diameter of 10 $\mu$m to 50 $\mu$m, and is particularly preferable to be a resin bead having a particle diameter of 15 $\mu$m to 30 $\mu$m.

<Reaction field>

In the present embodiment, the method for detecting a target substance includes: introducing a sample into a reaction field having a solid phase on which a second trapping substance is immobilized in advance; allowing the second trapping substance on the solid phase to react with a target substance in the sample; and then introducing a first trapping substance thereto, thereby forming a complex in which the target substance is sandwiched. After that, an excess first trapping substance is discharged out of the reaction system. Then, as described above, the bond of the complex is cleaved, and the moiety including the labeling substance is separated. The steps up to the separation of the moiety including the labeling substance are performed in the reaction field. It is possible to adjust a surface density of the second trapping substance to be fixed on the solid phase, in advance. For this reason, because of introducing the first trapping substance after the target substance has been reacted with the second trapping substance, it becomes possible to enhance the quantitative aspect of detection, in a wide range of the concentration of the target substance contained in the specimen, from a low concentration to a high concentration.

Note that, in the reaction for forming the complex in the reaction field, namely, in the step of mixing the first trapping substance, the solid phase on which the second trapping substance is carried, and a sample containing the target substance into the reaction field, they may be introduced in the reaction field in any order, and may be introduced in part or as a whole simultaneously, as long as the complex is formed.

In other words, the step of forming the complex is not limited to the step of forming the complex by reacting the target substance with the second trapping substance immobilized on the solid phase, and then reacting the resultant with the first trapping substance bound to the labeling substance, as described above. For example, it is acceptable to form the complex by reacting the target substance with the first trapping substance bound to the labeling substance, and then reacting the resultant with the second trapping substance immobilized on the solid phase, and it is also acceptable to form the complex by simultaneously reacting the first trapping substance bound to the labeling substance, the target substance, and the second trapping substance immobilized on the solid phase.

In addition, the reaction field is not particularly limited as long as it is a field in which it is possible to form the complex of the solid phase-the second trapping substance-the target substance-the first trapping substance (first trapping substance bound to labeling substance) and it is possible to separate the moiety including the labeling substance from the complex. In order to implement this reaction step, only one reaction field may be used, or a plurality of reaction fields may be used. In other words, the formation of the complex and the separation of the moiety including the labeling substance may be implemented in the same reaction field (space), or may be implemented in different reaction fields (spaces).

The reaction field may be formed with the use of, for example, a partitioned container or compartment. As the reaction field, it is possible to use, for example, a test tube, a microtube, a microplate, or a chamber or channel which is formed in a microreactor, a bio-disk or the like. In addition, it is acceptable to perform the operation in the reaction field manually or automatically. In the case where the operation in the reaction field is automated, it is acceptable to use, for example, a device in which the reaction field is mounted in a form of a chamber, and furthermore, it is acceptable that the device has a form of a disk which is rotatable around the central axis.

Preferably, the device includes at least one reaction field in a form of the reaction chamber. In the case where the device has a form of a disk, the reaction chamber is arranged closer to the central axis of the disk than the tubular channel, and communicates with the tubular channel in the outer side. The reaction chamber is usually formed so as to have a large size in both the depth direction and the width direction as compared with the inner diameter of the tube of the tubular channel. Preferably, the reaction chamber may be formed into such a shape (cone shape) as to be tapered toward the tubular channel. The moiety including the labeling substance in the liquid in the reaction chamber, which has been separated from the solid phase by the cleaving reagent, is intermittently supplied into the liquid with which the narrow tubular channel is filled, from the reaction chamber having a large capacity, when the disk rotates.

A communication port (inlet of tubular channel) through which the reaction chamber and the tubular channel communicate with each other needs to be formed in such a size as to cause the moiety including the labeling substance to pass therethrough and to prevent the solid phase from flowing out therethrough in the case where the solid phase formed as bodies separate from the reaction chamber is introduced into the reaction chamber (for example, in the case where the solid phase is a solid such as beads and has fluidity in the reaction chamber). In consideration of general processing accuracy, the communication port is for example, 5 $\mu$m or larger, is preferably 10 $\mu$m or larger, and is more preferably 15 $\mu$m or larger; but is designed so as to have a dimension which is easy to process, on the premise of the shape and size that do not allow the solid phase to flow into the tubular channel.

It is desirable that the moiety including the labeling substance which is the detection target is introduced into the detection field in a state in which foreign substances are reduced. In the method of the present invention, the labeling substance which has moved to the tubular channel by the centrifugal force, and accordingly is detected, when the excess labeling substance is mixed in the sample introduced into the tubular channel, the detection accuracy is greatly affected. Because of this, it becomes important to remove the excess first trapping substance (including the first trapping substance that binds to the labeling substance but fails to trap the target substance, and a released labeling substance) from a sample to be supplied to the tubular channel.

As a method for removing the excess first trapping substance from the sample, washing treatment may be selected. Specifically, if the washing treatment is performed after the complex in which the target substance is sandwiched has been formed, the excess first trapping substance which has not been involved in the reaction can be removed. On the other hand, the first trapping substance that has trapped the target substance is fixed to the solid phase via the target substance and the second trapping substance, and accordingly is retained in the reaction field. When the cleaving reagent is added in such a state, the separated moiety including the labeling substance can be introduced into the detection field under a condition that the excess first trapping substance has been removed to a level that does not affect the detection result.

In addition, as a method for removing the excess first trapping substance from the sample, it is acceptable to select centrifugal removal. It is possible to easily remove the excess first trapping substance by centrifugation with the use of a disk. Specifically, for example, when the complex is formed (or introduced) in the reaction chamber, the first trapping substance that has trapped the target substance is placed in the reaction chamber in a state of being fixed to the solid phase. On the other hand, the excess first trapping substance is not fixed, and accordingly, it is possible to move the excess first trapping substance to the tubular channel and discharge the excess first trapping substance to the outside of the reaction chamber by centrifugal force when the centrifugal force is imparted in a state in which the above tubular channel is filled with the liquid, the solid phase in the reaction chamber is immersed in the liquid, and the reaction chamber and the tubular channel communicate with each other through the liquid. After that, when the cleaving reagent is added to the reaction chamber to separate the moiety including the labeling substance from the complex, it is possible to introduce the moiety including the labeling substance into the detection field in a state in which the excess first trapping substance does not coexist. In this way, in the case where the excess first trapping substance is removed with the use of the centrifugal force, it is possible to eliminate the washing treatment with the use of the washing liquid such as a washing agent. Note that it is acceptable to perform the washing treatment with the use of both the removal of the excess first trapping substance by the centrifugal force and the washing with the washing liquid, from the viewpoint of enhancing the detergency.

Note that it is acceptable to retain a binding agent in the movement path of the excess first trapping substance, in order to fix the excess first trapping substance which has been discharged from the reaction chamber, with the binding agent which specifically binds to the first trapping substance. The binding agent is carried on a place which does not hinder the moiety including the labeling substance from passes through the tubular channel. Thereby, the excess first trapping substance which has been discharged from the reaction chamber is fixed at a predetermined place within the device.

As the binding agent, for example, such an antigen or an antibody is used as to specifically bind to the first trapping substance, or the same substance as the target substance of the first trapping substance may be used.

In addition to the above antigen and antibody, it is also acceptable to use a nucleic acid, an aptamer, a ligand (a substrate, an inhibitor or an antagonist) or a receptor, to any of which the first trapping substance binds, as the binding agent.

The antibody to be used as the binding agent may be a polyclonal antibody or a monoclonal antibody, but a monoclonal antibody is preferable, from the viewpoint of reaction specificity.

In addition, the antibody to be used as the binding agent includes: an antibody; and an antibody fragment and a modified antibody as well, which have substantially the same reactivity as the antibody. Examples of the antibody fragment include a Fab fragment, a F(ab')2 fragment, a Fab' fragment, and a scFv fragment.

<Detection field>

The detection field (second reaction field) is a field into which the moiety including the labeling substance separated in the reaction field (first reaction field) is introduced, and where scattered light caused by the moiety including the labeling substance is detected. In the detection field, the tubular channel is arranged in which the moiety including the labeling substance moves, and there are provided a light source for irradiating the tubular channel with irradiation light, and a light receiving element for detecting scattered light which is emitted from the labeling substance in the tubular channel.

In the above-described reaction field (first reaction field), if the washing treatment and centrifugation treatment described above are implemented after the complex has been formed, then elements other than the complex are discharged to the outside of the system. After that, if the moiety including the labeling substance is cleaved, then the sample is introduced into the detection field in a state in which the excess first trapping substance and other foreign substances that affect detection are reduced.

The tubular channel is provided in a device which is rotatably formed. The tubular channel is formed from a translucent material so that the irradiation light emitted from the light source reaches the inside thereof and the scattered light generated due to the detection target is radiated.

The tubular channel is preferably formed into a hollow cylindrical shape of which both ends are opened, and extends outward from the rotation center side along a rotation radius. An open end of the tubular channel on the rotation center side (centripetal direction side) (hereinafter, also referred to as "one open end") communicates with the reaction field (first reaction field) that retains a solution which contains the moiety including the labeling substance. As long as the tubular channel is a hollow tube, the cross-sectional shape thereof is not limited. The tubular channel may be a circular tube or a square tube, and may have a flat shape. In addition, the tubular channel may be formed of a single seamless part or may be formed of a combination of a plurality of parts.

An inner diameter of the tubular channel is designed to be in a range of about 3 times to 150 times the detection target so that clogging does not occur, and to have such a shape that the solid phase in the reaction field (reaction chamber) does not flow in. As described above, as for the communication port (inlet of tubular channel) with the reaction chamber, in the case where the solid phase is introduced into the reaction chamber, in other words, in the case where the solid phase is formed as bodies separate from the reaction chamber, the inner diameter of the tubular channel is configured to be smaller than the size of the solid phase, and, for example, to be a size of about 1/1.2 to 1/5 times the size of the solid phase. In detail, it is acceptable to set, for example, the smallest value of the respective maximum values of the length, width and height of the solid phase, as a reference value, and set the inlet diameter of the tubular channel so as to be smaller than the reference value. In the case where the solid phase is isotropic, the length, width and height are equal, and accordingly, any of these values may be used as the reference value. The inner diameter of the tubular channel may be formed uniformly with the same width as the inlet diameter, or may be formed with a width different from the inlet diameter, and for example, may be formed so as to become wide or narrow in a tapered shape or a stepped shape.

Specifically, for example, the inner diameter of the tubular channel is 1 $\mu$m to 100 $\mu$m, is preferably 5 $\mu$m to 50 $\mu$m, and is more preferably 10 $\mu$m to 20 $\mu$m. As the inner diameter is smaller, the amount (absolute amount) of foreign substances present in the tubular channel is reduced, and accordingly, it is possible to reduce a scattered light noise caused by foreign substances.

Note that in the case where the cross section of the tubular channel is an anisotropic shape such as an ellipse or a rectangle, the cross section is formed so that the shorter direction of the cross section becomes a height of the flow path of the tubular channel, and the longer direction becomes a width of the flow path. In addition, the tubular channel is designed so that the height direction becomes the above inner diameter.

When the height of the tubular channel is defined so that the solid phase does not flow into the tubular channel, the width of the flow path can be designed independently of the size of the solid phase. The minimum value of the width of the flow path of the tubular channel is a size of the moiety including the labeling substance, and the upper limit value is a width of the abutting surface of the reaction chamber communicating therewith. The wider the tubular channel becomes, the smoother the flow of the liquid in the tubular channel can be, but the abutting area against the reaction chamber expands; and as a result, the slope of the cone shape from the reaction chamber to the tubular channel becomes shallow, which may affect the accumulation range of the solid phase. From this point of view, a narrower width of the flow path of the tubular channel is preferred. Therefore, the width of the flow path of the tubular channel is set to an appropriate width in view of the flow of the liquid and the accumulation state of the solid phases in the reaction chamber; and for example, is 100 $\mu$m to 1 cm, is preferably 100 $\mu$m to 5 mm, is more preferably 500 $\mu$m to 3 mm, and is further preferably 500 $\mu$m to 1 mm.

An open end on the outer side of the tubular channel (hereinafter, also referred to as "the other open end") communicates with a drain reservoir serving as a second reservoir. The drain reservoir is a partitioned compartment for storing a liquid that has passed through the tubular channel. When the washing treatment is performed in the reaction field (first reaction field) which is formed in the device, the waste liquid of the washing treatment is discharged through the tubular channel and is stored in the drain reservoir. The drain reservoir may be disposed downstream from the tubular channel, and for example, a part thereof may be disposed on the rotation center side along the rotation radius with respect to the tubular channel, or the whole thereof may be disposed on the outer side.

Here, in the method of the present invention, it is necessary that the tubular channel be in a state of being filled with a liquid, in order to implement detection of the labeling substance. In other words, even in the case where the centrifugal force has been applied, it is necessary to ensure that the liquid in the tubular channel does not flow out of the open end. For this purpose, a restriction mechanism is provided which restricts a flow of the liquid in the tubular channel. This restriction mechanism can be realized by, for example, providing a thin flow path which extends from an end portion of the drain reservoir toward the rotation center side. According to this restriction mechanism, the liquid supplied to the tubular channel under an action of the centrifugal force is stored in the drain reservoir, and at the same time, the liquid that has overflowed progresses through the thin flow path toward the rotation center side. Here, a liquid surface is formed at the same distance from the rotation center, and accordingly, if the liquid is supplied until the liquid reaches the open end (the one open end) of the tubular channel on the rotation center side, also in the flow path toward the rotation center side, a liquid surface is formed at the same position as the open end (the one open end) of the tubular channel on the rotation center side. At this time, when the liquid receives the centrifugal force, even if the supply of the liquid is stopped, it is possible to suppress a return of the liquid to the upstream side. Thereby, the tubular channel results in being filled with the liquid.

In addition, it is acceptable to use the drain reservoir as the restriction mechanism. For example, in the case where at least a part of the drain reservoir is disposed on the rotation center side with respect to the tubular channel, it is acceptable to dispose the end portion on the side which does not communicate with the tubular channel so as to reach the open end(the one open end) on the rotation center side of the tubular channel, or so as to be closer to the rotation center side than the open end on the rotation center side of the tubular channel. When the liquid is supplied until the liquid reaches the open end (the one open end) on the rotation center side of the tubular channel, the liquid surface of the drain reservoir and the liquid surface of the tubular channel are formed to be the same distance from the rotation center. Thereby, the tubular channel results in being filled with liquid. Note that in this case, the drain reservoir acts to prevent the liquid in the tubular channel from flowing out from the open end, and it is not necessary to separately provide the restriction mechanism that restricts the flow of the liquid in the tubular channel.

Note that in the case where the washing treatment is not performed in the device, the drain reservoir may not be provided, and the thin flow path may be provided so as to extend from the end of the tubular channel toward the rotation center side.

In addition, as another example of the restriction mechanism, it is exemplified, for example, to provide an opening/closing valve at the other open end of the tubular channel or in a space to which the open end is connected. Thereby, when the opening/closing valve is closed, the other end of the tubular channel is closed, and accordingly, it becomes possible to fill the tubular channel with the liquid. Examples of the opening/closing valve include not only a valve which electrically, electromagnetically or mechanically operates, but also a capillary burst valve (a mechanism that functions as a valve because a liquid does not flow out from an open end until a centrifugal force exceeding a predetermined value is imparted) which will be described later; and an appropriate opening/closing valve can be selected from these examples, and be used.

At the time of detection, a centrifugal force is imparted in a state in which the tubular channel is filled with the liquid which has been supplied to the tubular channel, and thereby, the moiety including the labeling substance moves from the reaction field (first reaction field) located on the rotation center side to the outer side of the device, through the tubular channel.

Then, the moiety including the labeling substance which is the detection target is introduced into the tubular channel, and thereby, the irradiation light is scattered to generate scattered light. The scattered light is detected by the light receiving element such as a photodiode, and thereby, the target substance of interest are detected.

The intensity of the scattered light has a correlation with the particle diameter, in the case where the particle diameter of the labeling substance is within a predetermined range, and the intensity of the scattered light in a specific direction changes according to the particle diameter. The particle diameter of the particle to be used as the labeling substance is known in advance, accordingly, the light receiving element can be arranged at a position at which the scattered light can be detected, and the scattered light can be appropriately detected. Note that in the case where the scattered light is generated in all directions, the light receiving element can be arranged at an arbitrary position. The scattered light to be detected may be forward scattered light or may be backward scattered light. The light receiving element is appropriately arranged according to the direction in which the scattered light to be detected is scattered.

In addition, reflected light that becomes noise is generated at an interface on which the irradiation light is incident, but by adjusting the positions and angles of the light source and the light receiving element, respectively, it is possible to reduce the noise of the reflected light and to appropriately detect the scattered light emitted from the labeling substance.

Here, for example, if an optical system using such a general-purpose laser light source as to be used in an optical pickup, and a rotation mechanism are used, a small and inexpensive detection device can be realized. However, when the moiety including the labeling substance which is the detection target passes through the detection field at a high speed, it becomes difficult to accurately detect the scattered light based on the minute labeling substance, in a detection system which uses the inexpensive general-purpose member.

Here, in the present embodiment, in the case where a laser spot of the laser light source has a diameter of D $\mu$m, in the device rotating at N rpm (one rotation: 60/N seconds), the moiety including the labeling substance moves at a moving speed of v $\mu$m/s so that the labeling substance of the detection target enters the laser spot at least once. In this case, the following Expression holds.

$$v\frac{60}{N} \leq D \quad \cdots \cdots \quad (6)$$

When the Expression is solved for the moving speed v, the following Expression is obtained.

$$v \leq \frac{ND}{60} \quad \cdots \cdots \quad (7)$$

According to this Expression, for example, in the case where the spot diameter of the laser is 10 $\mu$m and the disk is rotated at 2000 rpm, the moving speed of the labeling substance needs to be 333 $\mu$m/sec or smaller.

As a method which is different from the method of the present invention, it is also considered to pass the moiety including the labeling substance through the tubular channel by a liquid flow and detect the scattered light emitted from the labeling substance which moves through the tubular channel. For this reason, the moving speed of the liquid which flows through the device by the centrifugal force has been calculated by the following Expression.

$$U = d_H{}^2\rho\omega^2 r_1\Delta r/32\eta L \quad ...(8)$$

wherein U is a flow velocity (m/s), Q is a flow rate (m$^3$/s), A is a cross-sectional area of the flow path (m$^2$), $d_H$ is a hydraulic diameter (m), $\rho$ is a density of fluid (kg/m$^3$), $\omega$ is an angular speed (rad/s), $r_1$ is an average rotation radius (m) of the liquid in the flow path, $\Delta r$ is difference in the rotation radius between the inlet and outlet of the flow path, $\eta$ is a viscosity of fluid (kg/m·s), and L is a length of the flow path (m).

In the case where a medium is water at 20°C and a disk is rotated at the number of rotations of 2000 rpm, a moving speed of the medium flowing by the centrifugal force, at a predetermined position in a radius direction on the disk, shall be calculated by the Expression (8), and a moving speed of the gold colloidal particle of 200 nm, which moves in the medium by the generated centrifugal force, shall be calculated by a Stoke's equation; and the former becomes 60 mm/sec and the latter becomes 84 $\mu$m/sec. In addition, it has been confirmed that the liquid in the disk actually moves at a high speed by the rotation. Therefore, in the case where the movement of the gold colloidal particle (labeling substance) is controlled by the flow of the liquid, it becomes difficult to detect the scattered light.

Then, in the present invention, the tubular channel is configured to be filled with a liquid so as to function as a centrifuge tube, and to cause the moiety including the labeling substance to move through the liquid by the centrifugal force, and to move in the tubular channel at such a moving speed that the scattered light can be detected.

In the present invention in which the movement of the moiety including the labeling substance in the tubular channel is not controlled by the liquid flow, the moving speed at which the moiety including the labeling substance moves in the tubular channel varies depending on the density and particle diameter of the moiety including the labeling substance (labeling substance) and the centrifugal force, if the liquid filled in the tubular channel is the same. Because of this, it is possible to set the moving speed in a desired range, by appropriately selecting the number of rotations of the device and the labeling substance.

<Device>

The device, which is called a bio-disk or a microreactor, includes at least a reaction chamber (corresponding to the above-described reaction field) for retaining the complex and, if necessary, performing the reaction, and a tubular channel (corresponding to the above-described detection field) for moving the detection target; and is optionally formed with the flow path having a minute diameter through which the liquid flows, and a reservoir for storing the reagent and the liquid; whereby it is used for analysis of a biological sample. In addition, the present device is formed so as to be rotatable for imparting the centrifugal force.

The present device can be produced by a known method, with the use of a known material including, for example, various plastics such as an acrylic resin, a polycarbonate resin and a silicone resin, and inorganic materials such as glass and silicon. It is preferable for the material to have translucency so that the detection target can be optically detected, and is more preferable to be transparent.

It is possible to produce the present device with the use of known techniques. It is possible to manufacture the present device by lamination of, for example, a sheet or plate having a pattern of a flow path or a chamber, and a flat sheet or plate. It is acceptable to form an internal structure of the reaction chamber and the like, by machining, etching with the use of photolithography, or other general molding techniques. Furthermore, a reflective layer may be arranged on a back surface of the tubular channel, in order to increase the amount of light of the signal incident on the light receiving element.

The reservoir which is provided in the present device and stores the reagent or the liquid is disposed on the rotation center side with respect to the reaction chamber, so as to supply the liquid to the reaction chamber by the centrifugal force. The method for detecting the target substance of the present invention includes moving the detection target in the liquid in the tubular channel by the centrifugal force, in a state in which the tubular channel is filled with the liquid. Because of this, in the case where the liquid to be filled in the tubular channel is stored in the reservoir in advance, the liquid stored in the reservoir fills in the tubular channel at a predetermined timing. In addition, in the present embodiment, in the case where the complex retained in the reaction chamber is subjected to the washing treatment, there are provided a washing liquid reservoir serving as the first reservoir and the drain reservoir serving as the second reservoir, and the washing liquid is stored in the washing liquid reservoir. The drain reservoir may be disposed on the downstream side of the tubular channel, at least a part of the drain reservoir may also be disposed on the rotation center side with respect to the tubular channel, and the whole may be disposed on the outer side with respect to the tubular channel. Note that the washing liquid can also serve as the liquid to be filled in the tubular channel, and in such a case, it is acceptable to form the above-described reservoir for storing the liquid to be filled in the tubular channel and the washing liquid reservoir, as one and the same chamber.

In the case where the tubular channel is filled with the washing liquid which is stored in the washing liquid reservoir, when the drain reservoir is, for example, at least partly disposed on the rotation center side with respect to the tubular channel, it is acceptable to dispose the drain reservoir so that the end portion on the side which does not communicate with the tubular channel is positioned at the same distance from the rotation center as the open end (the above one open end) located on the rotation center side of the tubular channel, or at a distance shorter than that. In addition, it is acceptable to store the washing liquid in the washing liquid reservoir at least in such a volume as to reach the open end located on the rotation center side of the tubular channel, in the drain storage reservoir and the tubular channel. In the case where the whole drain reservoir is disposed on the outer side with respect to the tubular channel, it is acceptable to store the washing liquid in the washing liquid reservoir in an amount exceeding the total inner capacity of the drain reservoir and the tubular channel, and less than the total inner capacity of the drain reservoir, the tubular channel and the reaction chamber. By such an amount of the washing liquid being stored, the tubular channel is filled with the washing liquid after the washing, and the liquid is also stored in the reaction chamber; and furthermore, it becomes possible that a remaining space of the reaction chamber receives the cleaving reagent for the moiety including the labeling substance.

In addition, in the case where the tubular channel is filled with the washing liquid which is stored in the washing liquid reservoir, the washing treatment may be performed until the drain reservoir and the tubular channel are filled with the washing liquid, and may be continued until the washing liquid exceeds the capacity of the tubular channel and flows into the reaction chamber, as long as the remaining space of the reaction chamber can receive the cleaving reagent for the moiety including the labeling substance. When the washing liquid flows into the reaction chamber in addition to the tubular channel so that the washing liquid is stored in the reaction chamber, the tubular channel and the reaction chamber communicate with each other through the liquid.

Furthermore, in the present embodiment, cleaving treatment for separating the moiety including the labeling substance from the complex is implemented, and in the case where the cleaving treatment is implemented in the device, a cleaving reagent reservoir serving as a third reservoir is provided, and a necessary cleaving reagent is stored therein. In addition, as described above, in the case where the sensitization reaction of the metal colloidal particle is performed, the sensitizing agent reservoir is provided, and the sensitizing agent solution necessary for the sensitization reaction is stored therein.

The reagent stored in such a reservoir may be stored in a state of a liquid or may be stored in a state of a solid. In the case where the reagent is stored in the solid state, it is configured that after a dissolving liquid for dissolving the reagent has flowed into the reservoir in which the reagent is stored, the reagent is sent from the reservoir to the reaction chamber.

Note that in the case where the cleaving reaction for separating the moiety including the labeling substance is implemented by energy being imparted from an external field such as heat or electromagnetic waves, it is acceptable not to install the cleaving reagent reservoir.

<Control system>

Each device which is arranged in the detection field for detecting the scattered light is electrically controlled by an information processing device. In addition, signal processing is implemented based on the received scattered light, and the labeling substance (moiety including labeling substance) is detected as a signal of scattered light.

The detection method of the present invention includes moving the labeling substance as the detection target in the tubular channel by centrifugal force generated by rotating the device. It is possible to obtain the moving speed, if the labeling substance, the liquid filled in the tubular channel, and the centrifugal force to be imparted are known. In addition, it is also possible to calculate an allowable moving speed of the labeling substance from the number of rotations and the spot diameter of the laser light source (the above Expression (7)). It is possible to derive a settable range of the rotation speed on the basis of the above factors.

The light receiving element detects the scattered light emitted from a position of a predetermined rotation radius. In other words, the position of the predetermined rotation radius is irradiated with the irradiation light emitted from the laser light source, and the spot diameter thereof is the detection area. The light receiving element is disposed at a position at which the scattered light emitted from the detection area is detected. Examples of the light receiving element include a photodiode, an avalanche photodiode, a photomultiplier tube, a CMOS and a CCD, but are not limited thereto.

When the labeling substance enters the spot diameter of the laser light source, the scattered light is incident on, for example, the photodiode serving as the light receiving element. Then, the incident light is photoelectrically converted and output from the photodiode, and a voltage value corresponding to the intensity of the incident scattered light is input to an A/D converter. Then, the input voltage values are sampled at predetermined sampling times by the A/D converter, and are thereby converted into digital signals, which are then input from the A/D converter to the information processing device. The A/D converter is not particularly limited as long as the A/D converter can convert an analog signal into a digital signal, and may be, for example, an integrated circuit or an oscilloscope. The sampling time is designed on the basis of resolution for the width of the flow path. The sampling time is set so that data can be acquired at intervals of, for example, 1/10 or longer and shorter than 1/1 of the spot diameter of the laser light source. For example, in the case where the tubular channel rotates at 2000 rpm and the spot diameter of the laser light source is 10 $\mu$m at a position of the rotation radius of 50 mm, the sampling time is set to about 100 ns in order to acquire data at intervals of about 1 $\mu$m.

In the information processing device, for example, immediately after the tubular channel is filled with the liquid and before the labeling substance is supplied to the tubular channel, blank data is stored in relation to a predetermined position in a width direction of the flow path of the tubular channel, as needed. After that, at every predetermined sampling time, the input voltage value is stored in relation to the same position, and the difference from the blank data is calculated. If the difference between the two is a predetermined threshold value or smaller, it is determined that the labeling substance is not detected at the position or that the scattered light is noise, and the generation of the scattered light is not counted (the counting is cancelled). On the other hand, in the case where the calculated difference exceeds a predetermined threshold value, it is determined that the scattered light is based on the labeling substance. Note that the labeling substance is smaller than the spot diameter of the laser light source, and accordingly, such a result is obtained that the scattered light is detected a plurality of times before the labeling substance passes through the spot diameter (detection area). Because of this, it may be configured that the number of times of generation of scattered light is counted as 1 when the voltage value has decreased (when the difference between the blank and the input voltage value has become a predetermined threshold value or smaller) after a plurality of measurements.

Furthermore, according to the detection method of the present invention, it is possible to detect the labeling substance (moiety including labeling substance) after the foreign substances have been sufficiently separated therefrom, but it can be assumed that, for some reason, foreign substances other than the labeling substance are mixed into the tubular channel at the timing of detecting the labeling substance (in a range of the predetermined time based on the moving speed of the labeling substance from the start of detection), or that the scattered light is generated due to the state of the tubular channel such as a flaw. In such a case, a signal pattern of the scattered light caused by a foreign substance or the tubular channel is different from a signal pattern of the labeling substance, and accordingly, both can be discriminated from each other; and the detection target of interest can be accurately detected. In the case where the detection target of interest can be identified and detected, it is not necessarily required to acquire the blank data before the labeling substance is detected as described above.

Furthermore, in the present detection method, it is possible to determine the labeling substance, the liquid to be filled in the tubular channel, and further, also the laser light source (spot diameter), in advance, and is also possible to derive the rotation speed (number of rotations) of the device, which is optimal for detection, in advance. Accordingly, it is possible to store these values in the information processing device by default. Note that it may be configured that a user inputs a necessary value as appropriate at the time of detection. Furthermore, in the case where it is known in advance that a unique scattered light is generated due to a flaw or the like of the tubular channel, it is acceptable to store the signal pattern as the blank data, by default.

<Detection method 1 for target substance (antibody test)>

An exemplary method 1 for detecting a target substance (antibody) according to the present embodiment will be described below. The present detection method is a method which has embodied the outline of the detection method described with reference to Figure 1, to an antibody test. In the present method, firstly, an antigen (second trapping substance) for the target substance is immobilized on the surface of a polystyrene bead (solid phase) according to an ordinary method. Then, after blocking has been performed, polystyrene beads (second trapping substance immobilized on solid phase) are introduced into a container having a predetermined volume. The polystyrene beads are introduced in a state of being dispersed in a buffer, then filtration, centrifugation or the like is appropriately implemented, and the solvent is removed. After that, a sample such as serum containing the target substance (antibody) is added, the sample is brought into contact with the beads for a certain period of time to react therewith; then, if necessary, the resultant beads are washed by the buffer, and subsequently, a secondary antibody solution (first trapping substance bound to labeling substance) is added; and the mixture is reacted for a certain period of time. Note that in this case, the labeling substance is assumed to be a metal colloidal particle. Thereby, an immunocomplex which has a sandwiched form of an antigen-antibody-secondary antibody is formed on the bead. After the beads have been washed with the buffer, a cleaving reagent (pH adjusting agent) is introduced so that the pH becomes 0 to 4 or 10 to 14, and the moiety including the metal colloidal particle is released from the immunocomplex formed on the solid phase.

Note that also in this antibody test, the user can arbitrarily select from which stage the reaction chamber of the device is used as the reaction field, after the antigen has been immobilized on a polystyrene bead (solid phase), as described above. In addition, in the case where the reaction chamber of the device is used, it may be determined that the washing treatment is performed only once after the immunocomplex of the antigen-antibody-secondary antibody has been formed on the polystyrene bead.

After the cleaving treatment, the separated moiety including the metal colloidal particle is detected. In the present method, the moiety including the metal colloidal particle separated by the cleaving treatment is introduced into the tubular channel of the device, and is detected. The reaction chamber connected to the tubular channel is provided with the communication port (inlet) which is formed with a height smaller than the diameter of the polystyrene bead, and the moiety including the metal colloidal particle is introduced or stored in the reaction chamber which is a chamber in front of the tubular channel. Then, the moiety including the metal colloidal particle is introduced into the tubular channel via the communication port (inlet), and then moves outwards in the tubular channel, by a centrifugal force. Here, because the tubular channel is filled with the liquid and the liquid is controlled so as not to substantially move, the moiety including the metal colloidal particle moves through this liquid phase by the centrifugal force. The tubular channel is irradiated with irradiation light, and scattered light is generated when the metal colloidal particle passes through the tubular channel. The scattered light is intermittently generated every time when the metal colloidal particle passes through. The signal corresponding to each metal colloidal particle is not obtained continuously but is obtained as a unique signal; accordingly, the metal colloidal particle can be detected by measuring this signal, and as a result, an antibody which is a target substance can be detected.

As described above, by such a method as above, a target substance detection method for detecting a target substance can be implemented.

<Detection method 2 for target substance (antigen test)>

An exemplary method 2 for detecting a target substance (antigen) according to the present embodiment will be described below. The present detection method is a further embodied method of the immunoassay which is called a sandwich method, the outline of which has been described with reference to Figure 1. In the present method, firstly, an antibody (second trapping substance) for the target substance is diluted to an appropriate concentration with the use of phosphate buffered saline (hereinafter referred to as PBS) or a buffer solution, and the antibody is immobilized on the surface of polystyrene bead (solid phase) according to an ordinary method. Then, after blocking, a predetermined volume of polystyrene beads (second trapping substance immobilized on solid phase) is filled in a container having a predetermined volume. A sample such as serum containing the target substance (antigen) is added thereto, and the sample is brought into contact with the beads for a certain period of time to react therewith; then the beads are washed with a buffer; and subsequently, a primary antibody which is labeled with a metal colloidal particle (first trapping substance bound to labeling substance) is added, and is bound to the antigen. Thereby, an immunocomplex which has a sandwiched form of an antibody-antigen-antibody is formed on the styrene bead.

Note that also in the present method, the user can arbitrarily select from which stage the reaction chamber of the device is used, as in the above detection method 1. In addition, in the case where the reaction chamber of the device is used, it may be determined that the washing treatment is performed only once after the immunocomplex of the antibody-antigen-antibody has been formed on the polystyrene bead. The detection of the moiety including the metal colloidal particle is the same as in the detection method 1 described above, and thereby, an immunoassay for detecting the target substance can be implemented.

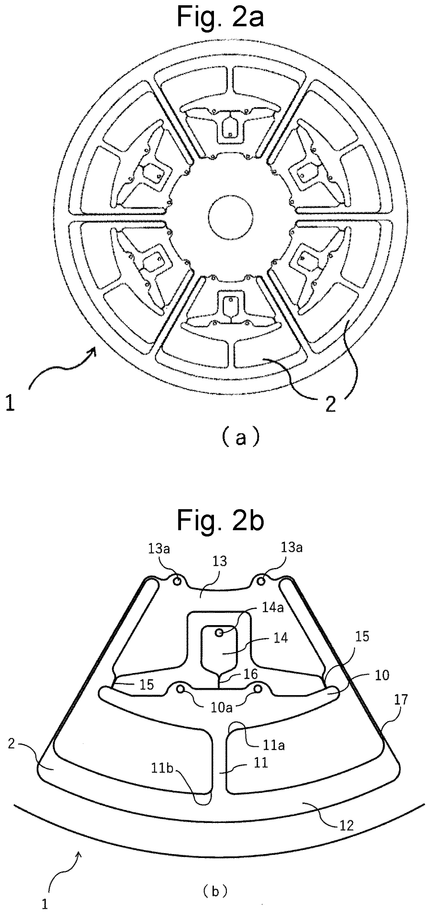

An example of the device of the present embodiment will be described in more detail below with reference to Figure 2. Figure 2 is a view showing an outline of a disk 1 for implementing the detection of the target substance; and Figure 2(a) shows an overall view of the disk, and Figure 2(b) shows a partially enlarged view thereof. The present disk 1 is a discoid plastic disk such as a compact disc (CD), a DVD, or a Blu-ray Disc (BD), in which flow paths each having a minute diameter through which a liquid can flow and rooms are formed. In order to impart the centrifugal force, a disk shape suitable for rotation is adopted, and the center of the circular plate becomes the center of rotation. The disk 1 is provided with six units 2, and is configured so that each unit 2 can independently detect a target substance.

Each unit 2 includes: a reaction chamber 10 in which the complex is retained; a tubular channel 11 which is connected to the reaction chamber 10; a drain reservoir 12 which is connected to the tubular channel 11; a washing liquid reservoir 13 which stores the washing liquid therein; and a cleaving reagent reservoir 14 which stores the cleaving reagent therein.

The reaction chamber 10 has, on its upper surface, a supply port 10a which penetrates between the inside and outside of the chamber, and is formed so that solutions containing solid phases, reagents and the like can be charged from the supply port 10a. The reaction chamber 10 serves as the reaction field in the present disk 1. When the centrifugal force is imparted to the solid phase which has been introduced into the reaction chamber 10, the solid phase accumulates on an inner wall (including all of upper surface, bottom surface, and side surfaces) on the side of the tubular channel 11. Here, the outer side of the reaction chamber 10 is formed in a mortar-like shape seen in a top view so that the solid phases accumulate while being prevented from spreading in the direction of width (length in a direction intersecting rotation radius) of the reaction chamber 10. In addition, in the inside of the reaction chamber 10 on the side of the tubular channel 11, the bottom surface is formed so as to be obliquely inclined so that the bottom becomes gradually shallower toward the tubular channel 11 side.

The present disk 1 is configured so that necessary processes can be sequentially implemented according to the steps shown in Figure 1. In the present disk 1, a process of separating the moiety including the labeling substance from the complex can be performed in the reaction chamber 10; and in the reaction chamber 10, the formed complex (that is one composed of the solid phase-second trapping substance-target substance-first trapping substance bound to a labeling substance, and the like) is retained.

The tubular channel 11 is a hollow tube having both ends opened, and extends along the rotation radius of the disk 1; and also, the opening end of the tubular channel 11 on the side of the center of rotation forms a communication port (inlet) 11a which communicates with the reaction chamber 10, and the other opening end forms a communication port (outlet) 11b which communicates with the drain reservoir 12. This tubular channel 11 is formed from a translucent material. The width of the flow path of the tubular channel 11 is formed so as to be narrower than the width of the reaction chamber 10. In addition, the depth thereof is formed to be smaller than the minimum diameter of the outer shape of the solid phase to be introduced so that the solid phase introduced into the reaction chamber 10 can be blocked. In the present disk 1, the tubular channel 11 abuts on the upper side of the reaction chamber 10, and communicates with the reaction chamber 10. The tubular channel 11 is configured so as to dam the solid phase introduced into the reaction chamber 10 by defining the depth direction thereof, and accordingly, the width of the flow path is designed regardless of the solid phase size.

The drain reservoir 12 is continuously disposed outwards of the tubular channel 11 in the direction of rotation radius of the disk 1, and stores the waste liquid generated in the reaction chamber 10. The tubular channel 11 and the drain reservoir 12 communicate with each other through the communication port (outlet) 11b, and the waste liquid in the reaction chamber 10 is discharged to the drain reservoir 12 via the tubular channel 11. In addition, the drain reservoir 12 is developed to both sides of the communication port 11b, and is connected to thin flow paths 17 which extend from both of the above ends toward the rotation center side. The flow path 17 communicates with the washing liquid reservoir 13 at an ultimate end thereof. The liquid supplied in excess of the capacity of the drain reservoir 12 enters the flow path 17 and also enters the tubular channel 11.

The washing liquid reservoir 13 is a partitioned compartment which stores the washing liquid to be added to the reaction chamber 10, and has an inlet 13a which is penetrated for introducing the washing liquid therethrough, on the upper surface side of the disk. In addition, the washing liquid reservoir 13 is arranged on the rotation center side with respect to the reaction chamber 10, and is connected to the reaction chamber 10 by a flow path 15. Note that in the unit 2, only one washing liquid reservoir 13 is provided, and the washing liquid for one time is stored therein. In the present disk 1, the washing liquid reservoir 13 stores the washing liquid in an amount exceeding the total inner capacity of the reservoir 12 and the tubular channel 11 and less than the total inner capacity of the reservoir 12, the tubular channel 11 and the reaction chamber 10.

The cleaving reagent reservoir 14 is a partitioned compartment for storing the cleaving reagent which is added to the reaction chamber 10, and has an inlet 14a which penetrated for introducing the reagent therethrough, on the upper surface side of the disk. In addition, the cleaving reagent reservoir 14 is arranged on the rotation center side with respect to the reaction chamber 10, and is connected to the reaction chamber 10 by a flow path 16.

The cleaving reagent which has been adjusted to a high concentration is accommodated in the cleaving reagent reservoir 14 so as to become a desired final concentration in the case where the whole amount of the cleaving reagent stored in the cleaving reagent reservoir 14 has been added to the reaction chamber 10.

The flow paths 15 and 16 are each a hollow pipe which has a self-opening and closing minute diameter with openings at both ends. Here, the "self-opening and closing" indicates a state in which it is possible to control movement of a fluid into and out of the flow path. Specifically, the opened state and the closed state can be switched according to the number of rotations. Note that in the present embodiment in which the number of rotations is increased step by step, once it is in the opened state, the flow paths 15 and 16 remain opened until a series of processes is completed; and the flow paths 15 and 16 have a function of self-opening and closing, but are formed so as to be substantially openable. In other words, in the detection method of the present invention, it is acceptable to allow the cleaving reagent reservoir 14 and the reaction chamber 10 to communicate with each other through an openable flow path, in place of the self-opening and closing flow path. The flow paths 15 and 16 have each a function called a capillary burst valve; and are configured to become an open mode when being imparted with the centrifugal force of a predetermined value or larger, and are configured so that the fluid does not flow when the centrifugal force is the predetermined value or less. The inner diameter of the flow path 15 is designed so as to be larger than the inner diameter of the flow path 16, and the flow path 15 is opened at a lower centrifugal force (lower number of rotations) than that at which the flow path 16 is opened. Thereby, the washing liquid is supplied to the reaction chamber 10 from the washing liquid reservoir 13. After that, when the number of rotations is increased, the flow path 16 is opened, the cleaving reagent in the cleaving reagent reservoir 14 flows, and the cleaving reagent is added to the reaction chamber 10.

Note that the number of rotations at which the washing liquid is supplied to the reaction chamber 10 is determined on the basis of the easiness of the burst. The easiness of the burst varies depending on a shape of the flow path and properties of the liquid which flows through the flow path. For example, it is influenced by a cross-sectional area of the flow path, a length of the flow path, position (rotation radius) and shape of the inlet and outlet of the flow path, and the surface tension, contact angle and viscosity of the liquid. Accordingly, the number of rotations is selected theoretically or experimentally in consideration of these factors. Note that it is desirable that the number of rotations be set preferably at 10,000 rpm or smaller, in view of a mechanical load.

The present disk 1 is configured so that the number of washing times is once, but when the number of washing times is plural, the washing liquid reservoir 13 is provided to be divided into plural sections, and flow paths are provided in the sections, respectively. In addition, each flow path is connected to the reaction chamber 10. In addition, the respective flow paths are designed to open at lower number of rotations than the number of rotations at which the flow path 16 opens, and also at different numbers of rotations, respectively.

In the case where the target substance is detected with the use of this disk 1, firstly, a solution in which beads (solid phases) are dispersed on each of which the second trapping substance is solid-phased is introduced into the reaction chamber 10 from the supply port 10a. In the reaction chamber 10, a communication port 11a which communicates with the tubular channel 11 is formed, and the solid phase which has such a size as not to pass through the communication port 11a is selected. Accordingly, the solid phase in the introduced solution is retained in the reaction chamber 10.

Next, a sample which has been diluted to an appropriate concentration is supplied to the reaction chamber 10 from the supply port 10a so that the target substance in the sample is trapped by the second trapping substance on the bead. After a predetermined time has elapsed, when a solution containing the first trapping substance is supplied to the reaction chamber 10 from the supply port 10a, the first trapping substance binds to the target substance, and the complex is formed. Thereby, the complex is retained in the reaction chamber 10. Note that a solvent in the reaction chamber 10 is discharged from the communication port 11a along with the rotation, and is stored in the drain reservoir 12 through the tubular channel 11.

When the disk 1 is rotated at a predetermined number of rotations so that the flow path 15 is opened, the washing liquid in the washing liquid reservoir 13 is supplied to the reaction chamber 10, and washing is implemented. Thereby, the excess first trapping substance and other foreign substances in the reaction chamber 10 are transferred to the drain reservoir 12 by the washing liquid.

As the washing liquid is discharged from the washing liquid reservoir 13, the washing liquid is gradually stored in the drain reservoir 12. Because the washing liquid exceeding the inner capacity of the drain reservoir 12 is stored in the washing liquid reservoir 13, the washing liquid overflows from the drain reservoir 12 and enters the flow path 17, and furthermore, the liquid level rises also in the tubular channel 11. Here, the centrifugal force toward the outside works on the disk 1, and accordingly, it does not occur that the washing liquid results in disorderly flowing to the reaction chamber 10 side. In addition, the flow path 17 serves as an air vent, and the air in the reaction chamber 10, the tubular channel 11 and the drain reservoir 12 is expelled to the flow path 17, and is pushed out to the washing liquid reservoir 13. When all the washing liquid is discharged from the washing liquid reservoir 13, the drain reservoir 12 and the tubular channel 11 are filled with the liquid, and furthermore, the liquid accumulates also in the reaction chamber 10; and such a state is formed that the solid phase is immersed while an airspace is left on the rotation center side. Note that most of the first trapping substances transferred to the drain reservoir 12 by the flow of the washing liquid sediment to the bottom surface (outer side) of the drain reservoir 12, by the centrifugal force acting thereon.

Next, the number of rotations is increased to a predetermined number of rotations, at which the flow path 16 is opened, and the cleaving reagent stored in the cleaving reagent reservoir 14 is caused to flow out to the reaction chamber 10. Thereby, the concentration of the cleaving reagent in the reaction chamber 10 gradually increases, and the moiety including the labeling substance is cleaved and separated from the complex. The separated moiety including the labeling substance moves to the drain reservoir 12 through the tubular channel 11.

A predetermined position of the tubular channel is irradiated with irradiation light emitted from a light source. Because of this, when the moiety including the labeling substance passes through the tubular channel 11, scattered light is generated. By detecting the scattered light using the light receiving element, the moiety including the labeling substance can be appropriately detected, and as a result, the target substance can be detected.

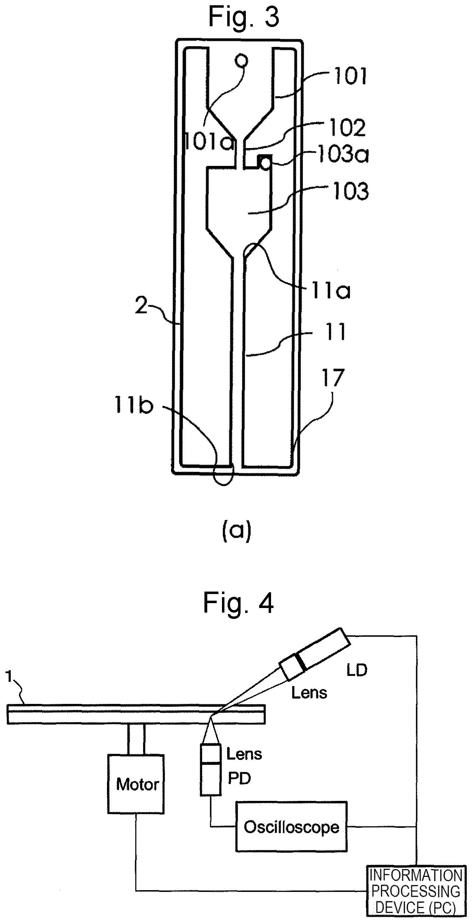

Figure 3 shows a modified example of the disk 1 according to the first embodiment, and is a view showing another example of the disk. In the present modified example, specifically, the unit 2 of the disk 1 is changed, and in Figure 3, the unit 2 of the present modified example is shown; and the same parts as those of the above-described disk 1 are denoted by the same reference numerals, and the description thereof will be omitted.

In the present unit 2, there are provided a first reservoir 101 for retaining the sample therein, a flow path 102 which communicates with the first reservoir 101, a second reservoir 103 which stores the liquid to be filled in the tubular channel 11 and the tubular channel 11, from the rotation center side toward the outside; and furthermore, the thin flow path 17 which extends from an end portion of a branch flow path which is branched from a leading end of the tubular channel 11 toward the rotation center side is provided.

The present unit 2 is a unit for introducing, into the disk 1, a sample which has been subjected to the separation of the moiety including the labeling substance in a reaction field outside the disk 1, and for detecting the moiety including the labeling substance. Because of this, the washing treatment is not implemented in the disk 1.

In the present unit 2, firstly, a predetermined amount of the sample solution is introduced into the first reservoir 101 from an opening 101a of an upper surface of the first reservoir 101. Here, because the flow path 102 is configured to have a function of the capillary burst valve, the sample solution introduced therein in a state of standing still does not flow out to the second reservoir 103 through the flow path 102. In addition, a predetermined amount of a liquid to be filled in the tubular channel 11 is introduced into the second reservoir 103 from an opening 103a of an upper surface of the second reservoir 103. The second reservoir 103 is provided at the end portion on the rotation center side with an introduction portion which is provided at a position deviated from the extension of the flow path 102 and protrudes toward the rotation center side. The opening 103a is formed by penetrating the upper surface of the introduction portion. Thereby, when the sample solution which is retained in the first reservoir 101 is introduced into the second reservoir 103, it is possible to prevent the liquid from leaking from the opening 103a.

The tubular channel 11 is structured so as to abut on an upper portion in the depth direction of the second reservoir 103 and communicate therewith. In the second reservoir 103, a necessary amount of the liquid for filling the tubular channel 11 is stored; but, in order that the tubular channel 11 is filled with the liquid, the tubular channel 11, the branch flow path ahead of the tubular channel 11, and the flow path 17 communicating with the branch flow path up to a position corresponding to the communication port (inlet) 11a of the tubular channel 11 result in being filled with the liquid; and accordingly, the second reservoir 103 is designed on the basis of the total internal volume of the tubular channel 11, the branch flow path ahead of the tubular channel 11, and the above corresponding portions of the flow path 17. In addition, the structure of the second reservoir 103 is designed so that the liquid surface of the stored liquid is lower than the bottom surface of the communication port 11a of the tubular channel 11 in a state in which the liquid is stored therein. Note that when the device 1 is formed from a general-purpose resin material, the insides of the first and second reservoirs 101 and 103 have hydrophobic surfaces, and accordingly, tend to act to keep the water base sample solution and the liquid for filling the tubular channel which have been introduced, in the respective compartments.

Then, the device 1 is rotated at a predetermined number of rotations, at which the flow path 102 is not opened, and thereby, the liquid in the second reservoir 103 is introduced into the tubular channel 11. Here, because the flow path 17 functions as the air vent, when the disk 1 is rotated at a predetermined number of rotations, the liquid in the second reservoir 103 is smoothly introduced into the tubular channel 11.

Subsequently, when the rotation speed is increased to the number of rotations at which the flow path 102 is opened, the sample solution flows out to the second reservoir 103 from the first reservoir 101. Then, the moiety including the labeling substance moves outwards in the tubular channel 11 by the centrifugal force. In this way, in the case where the cleaving treatment is implemented in the reaction field outside the disk 1, it is possible to implement the detection of the target substance with a simple configuration of the device.

Note that an apparatus shown in Figure 4, for example, is used for the rotation of the disk 1. The apparatus includes a motor (Motor) and a rotation shaft connected to the motor, and a mounting table for rotating the disk 1 in a horizontal direction is axially supported by the rotation shaft. When the motor is driven, the disk 1 which is placed on the upper surface of the mounting table rotates, and the centrifugal force is imparted. The motor is connected to an unillustrated microcontroller, and is connected to an information processing device (personal computer (PC)) via the microcontroller. The information processing device transmits and receives signals to and from each connected device, and controls each device. Note that the information processing device (PC) is not particularly limited as long as the device can implement signal control. When a control signal transmitted from the information processing device is input to the microcontroller, the microcontroller controls the motor so as to rotate at a predetermined number of rotations, which has been determined beforehand, for a predetermined time period.

The mounting table is provided with a window which penetrates in the thickness direction, at a position facing the tubular channel 11 in the case where the disk 1 has been mounted. Above the mounting table, a laser light source (LD) which has been adjusted so that irradiation light is incident at an oblique angle with respect to the upper surface of the mounting table, is installed. In front of the laser light source, a focus lens (Lens) is disposed, and is adjusted so that the irradiation light emitted from the laser light source is focused on the inside of the tubular channel 11. To this laser light source, the PC is connected via the above microcontroller, and the output is controlled by the microcontroller which has received an instruction from the PC.

Below the window of the mounting table, a condensing lens (Lens) and a photodiode (PD) are arranged so as to receive the scattered light. The intensity of the scattered light is converted into a voltage by the photoelectric conversion by the photodiode, and input to a signal detector (Oscilloscope). In the signal detector, sampling is implemented at a predetermined sampling time, and the input analog signal (voltage value) is converted into a digital signal, and is input to the PC. Then, the measurement result of the scattered light is output in the PC. Thereby, it is possible to acquire the scattered light of the labeling substance, and to detect the target substance.

In the above, the present invention has been described based on the embodiments, but the present invention is not limited to the above embodiments, and it can be easily inferred that various improvements and modifications are possible within a range which does not deviate from the gist of the present invention.

For example, the disk 1 may be configured so as to be appropriately provided with a mechanism for controlling the movement of the fluid. Examples of such a mechanism include a valve. Such a valve is a valve which switches the communication port of the flow path or the compartment between the closed state and the opened state, and various types of valves which are operated by electricity, a magnetic force, an electromagnetic force, a mechanical mechanism, an inertia force, a centrifugal force, and heat can be used, in addition to the capillary burst valves provided in the above flow paths 15 and 16.

Furthermore, for example, the disk 1 has been described as a circular plate, but is not limited to a circular plate in the present invention, and the disk 1 may have another shape. In other words, in the method of the present invention, the shape of the device is not particularly limited as long as the device is embodied to be capable of imparting the centrifugal force. Because of this, for example, a chip type of device which includes only one unit 2 can be exemplified, and has an outer shape formed in a fan shape or a rectangular shape. In such a case, it is possible to provide a mechanism or a jig for retaining the chip on the mounting table of the apparatus for rotating the device, and rotate it with the chip being engaged therein to impart the centrifugal force.

As a modified example of the present invention, the following invention can be exemplified.

A method of detecting a target substance is shown, which includes: bringing a binding agent that specifically binds to a first trapping substance into contact with a sample in which a complex that has been formed by the target substance which has been trapped by the first trapping substance that is bound to a labeling substance, and the first trapping substance that does not trap the target substance are mixed; thereby binding the first trapping substance that does not trap the target substance, to the binding agent; placing the binding agent in a chamber in which a sample is stored; imparting the centrifugal force, leading the complex out of the chamber, and moving the complex in a tubular channel filled with a liquid; and detecting the complex by the scattered light which is generated by the complex which moves in the tubular channel and scatters irradiation light.

In the present modified example, the detection target is the complex which has been formed by binding of the target substance and the first trapping substance. Here, the excess first trapping substance which has not been bound to the target substance is also bound to the labeling substance, and accordingly, if this is introduced into the detection field, the scattered light results in being detected also from an unreacted first trapping substance, and an error occurs in the detection result. In the above described first embodiment, the excess first trapping substance is removed in a way that the target substance is immobilized on the solid phase, and the first trapping substance is once carried on the solid phase; and in addition, by an appropriate washing treatment; but in the present modified example, the excess first trapping substance is bound to the binding agent, and the binding agent is retained in the chamber; and accordingly, it is possible to retain the excess first trapping substance in the chamber, and it becomes possible to attempt the enhancement of the accuracy of the complex detection while suppressing the introduction of the excess first trapping substance into the detection field and eliminating the need for washing.

The formation of the complex and the binding of the excess first trapping substance to the binding agent are implemented in a state where the liquid is retained. For example, a tubular channel and a front chamber (chamber) for supplying the sample to the tubular channel are provided in one device, and the binding agent is retained in the front chamber; and then it is possible to bind the excess first trapping substance contained in the sample which has been introduced into the front chamber, to the binding agent, and to retain the resultant in the front chamber. Note that the chamber in which the binding agent is retained is not necessarily formed integrally with the device, and another chamber provided outside the device may be used for retaining the excess first trapping substance. In the case where the excess first trapping substance is retained in the chamber, it is possible to appropriately select a mode in which the binding agent is fixed to an inner wall of the chamber, or a mode in which the binding agent is fixed to a solid and introduced into the chamber.

In addition, in the case where the binding agent is introduced into the front chamber, if the solid to which the binding agent is fixed is formed larger than the tubular channel, it is possible to retain the excess first trapping substance in the front chamber. Furthermore, it is acceptable to configure the chamber to be provided with a valve, to open the valve after the liquid has completely filled the tubular channel, and to introduce the complex formed by the binding of the target substance and the first trapping substance into the tubular channel.

<Binding agent>

The binding agent is an agent which specifically binds to the first trapping substance, and an antigen is preferably exemplified. In addition, the antigen may be directly carried on a solid phase such as an inner wall or a solid, or may be indirectly fixed to a solid phase via an antibody or the like. In addition, for example, in the case where an antigen is used as the binding agent, if a tag such as biotin is attached to the antigen and avidin is bound to the solid phase, it is possible to immobilize the binding agent on the solid phase by binding of biotin and avidin.

Note that for the binding between the solid phase and the binding agent, it is possible to use binding modes, for example, such as physical adsorption, covalent bonding, ionic bonding, binding which utilizes affinity, and combinations thereof. Preferably, the binding agent is immobilized on the solid phase by covalent bonding or ionic bonding, which is stronger than a binding force due to affinity or adsorption.

Note that in the case where the labeling substance is larger than the first trapping substance, it is assumed that a plurality of first trapping substances are bound to the labeling substance, but in this modified example, the smaller the number of the first trapping substances bound to the labeling substance is, the better the detection is realized, and ideally, it is desirable that the number of the first trapping substances bound to the labeling substance is one.

Examples

The present invention will be described in more detail below with reference to Examples. Note that the present invention is not limited to the description of the Examples.

(Reference Example 1) Preparation of platinum-gold colloidal suspension

All glassware to be used was washed with aqua regia. In a flask, 390 mL of ultrapure water was charged, and was boiled; to this boiling water, 30 mL of an aqueous solution of chloroauric acid (1 g as gold per liter of aqueous solution, manufactured by Katayama Chemical Industries, Co., Ltd.) was added; and then 60 mL of a 1 wt% aqueous solution of sodium citrate was added. After the addition of the aqueous solution of sodium citrate, several tens of mL of aqueous solution of chloroplatinic acid (1 g as platinum per liter of aqueous solution, manufactured by Fujifilm Wako Pure Chemical Corporation) was added. Furthermore, after 5 minutes, 60 mL of the 1 wt% aqueous solution of sodium citrate was added, and the mixture was refluxed for 4 hours to obtain a platinum-gold colloidal suspension. Note that in order that the moiety including the labeling substance can be accurately detected, the particle diameter of the colloid was adjusted to be 100 nm or larger by appropriate addition of a reagent or a reducing agent.

(Reference Example 2) Preparation of monoclonal antibody

Commercially available SARS-CoV-2 nucleocapsid protein (hereinafter, simply referred to as "SARS-CoV-2NP") (CUSABIO Company) was used as an antigen for immunization, and anti-SARS-CoV-2NP monoclonal antibody (hereinafter, simply referred to as "anti-NP antibody") was obtained according to a conventional method.

Specifically, a mouse (BALB/c, 5 weeks old, Nippon SLC) was immunized three times with 100 $\mu$g of SARS-CoV-2NP, and the spleen cells were used for cell fusion. For the cell fusion, an Sp2/0-Ag14 cell (Shulman et al., 1978) was used, which is a myeloma cell of a mouse. For culturing the cells, a culture solution which was prepared by adding 0.3 mg/ml of L-glutamine, 100 unit/ml of potassium penicillin G, 100 $\mu$g/ml of streptomycin sulfate, and 40 $\mu$g/ml of Gentacin (DMEM), to Dulbecco's Modified Eagle Medium (Gibco), and then adding fetal bovine serum (JRH) to the resultant mixture so that the concentration becomes 10%, was used. The cell fusion was performed by mixing the spleen cell of the immunized mouse with Sp2/0-Ag14 cell, and adding a polyethylene glycol solution (Sigma) thereto. The fused cells were cultured in HAT-RPMI [serum-added RPMI containing 0.1 mM of Sodium Hypoxantine, 0.4 $\mu$M of Aminopterin and 0.1 mM of Thymidine (Gibco)], and the production of antibodies in the culture supernatant was confirmed by enzyme-linked immunosorbent assay (ELISA). The antibody-producing positive cells were cultured and grown in HT-RPMI [serum-added RPMI containing 0.1 mM Sodium Hypoxantine and 0.1 mM Thymidine].

The cloned cells were intraperitoneally inoculated into mice (BALB/c, retired, Japan SLC) which had been inoculated with 2,6,10,14-Tetramethylpentadecane (Kishida Chemical Co., Ltd.), and the ascites was collected. The ascites was supplied to a protein G column, and the monoclonal antibody was purified. Finally, 83 clones of monoclonal antibody-producing cells against SARS-CoV-2NP were obtained.

(Reference Example 3) Preparation of gold colloidal suspension

Gold colloidal particles were prepared with the use of an aqueous solution of chloroauric acid (1 g as gold per liter of aqueous solution, manufactured by Katayama Chemical Industries, Co., Ltd.) as a raw material, according to a conventional method. Specifically, 1 ml of a 1% (v/w) aqueous solution of chloroauric acid was added to 99 ml of pure water boiled by heating, and furthermore, after 1 minute, 1.5 ml of a 1% (v/w) aqueous solution of sodium citrate was added thereto; and the mixture was boiled for 5 minutes by heating, and then was cooled by being left at room temperature. Subsequently, to this solution, 200 mM of an aqueous solution of potassium carbonate was added to adjust the pH 9.0; and to the mixture, ultrapure water was added so that the total amount became 100 ml, and thereby a gold colloidal suspension was obtained in which gold colloidal particles were suspended. Note that in the case where a desired particle diameter was not obtained, the amount of the reducing agent to be added to chloroauric acid or the like was appropriately adjusted, and gold colloidal particles were produced which had a necessary particle diameter.

(Example 1) Separation of moiety including labeling substance (platinum-gold colloidal particle) by pH adjusting agent

(1) Preparation of platinum-gold colloid-labeled antibody solution

One anti-NP antibody selected from the anti-NP antibodies which were obtained in Reference Example 2 was labeled with platinum-gold colloid according to the following procedure. The anti-NP antibody in an amount of 1 $\mu$g in terms of protein weight (hereinafter, when weight in terms of protein weight is indicated, the weight is indicated by weight value by gravimetric analysis of the purified protein) was mixed with 1 mL of the platinum-gold colloidal suspension having a particle diameter of 120 nm, which was prepared in Reference Example 1, and the mixture was allowed to stand still at room temperature for 2 minutes; and thereby, the anti-NP antibody was bound to the surface of the platinum-gold colloidal particle. After that, a 10% aqueous solution of bovine serum albumin (hereinafter, referred to as "BSA") was added thereto so that the final concentration became 1.0% with respect to the total liquid amount, and the remaining surface of the platinum-gold colloidal particles was blocked with BSA; and thereby, a platinum-gold colloid-labeled anti-NP antibody solution was prepared. After that, this solution was centrifuged (600$\times$g, for 25 minutes) to precipitate the platinum-gold colloid-labeled anti-NP antibody, and the supernatant was removed; and thereby, the platinum-gold colloid-labeled anti-NP antibody was obtained. The obtained platinum-gold colloid-labeled anti-NP antibodies were suspended in 50 mM tris-hydrochloric acid buffer solution (pH 7. 4) containing 10% saccharose, 1% BSA, and 0.5% Triton-X100 (trade name), and thereby a platinum-gold colloid-labeled antibody solution was prepared.

(2) Cleaving treatment with pH adjusting agent

An antibody dilution solution was produced by adjusting an antibody other than the anti-NP antibody which was used for the previously described platinum-gold colloid-labeled anti-NP antibody among the anti-NP antibodies obtained in Reference Example 2, to 20 $\mu$g/mL with a tris-hydrochloric acid buffer solution; 100 $\mu$L of the antibody dilution solution was added to each well of a 96-well microplate, and the plate was allowed to stand still for 15 to 18 hours; and the antibody was solid-phased. After that, blocking treatment was performed; and then, 50 $\mu$L of a SARS-CoV-2NP solution adjusted to 10 ng/mL and 50 $\mu$L of a platinum-gold colloid-labeled antibody solution were added thereto, and the mixture was allowed to stand still at 37°C for 10 minutes. Subsequently, the wells were washed with TBS-T (Tris Buffered Saline with Tween 20), and after the washing liquid was discharged, 100 $\mu$L of various reagents having different pHs were added; and the wells were allowed to stand still at room temperature for 2 minutes, and then the solutions in the wells were collected. The types and the pH values of the reagents which were used are shown in Table 1. The pH value was measured with the use of a pH meter (LAQUA, manufactured by Horiba, Ltd.).

Note that a pH of a citric acid solution was adjusted to a desired pH (pH 2.94 to pH 6.96) by mixing citric acid and trisodium citrate. In addition, phosphoric acid solutions were prepared by mixing sodium dihydrogen phosphate and disodium hydrogen phosphate so that the pH of the mixture became a desired pH in the vicinity of neutrality (pH 6.99 to 8.56), and by mixing disodium hydrogen phosphate and trisodium phosphate so that the pH of the mixture became a desired pH of alkalinity (pH 11.33 to 12.85). A glycine hydrochloride buffer solution, a glycine NaOH buffer solution, and a triethylamine solution were adjusted to a desired pH by addition of hydrochloric acid or sodium hydroxide. In addition, pHs of the hydrochloric acid solution and the sodium hydroxide solution were adjusted by changing a dilution ratio.

Table 1

| pH Adjusting agent | pH Value | | | | |
|---|---|---|---|---|---|
| Hydrochloric acid | 1.89 | | | | |
| Glycine-HCl | 1.53 | 2.48 | 3.18 | | |
| Citric acid | 2.94 | 3.97 | 4.92 | 5.93 | 6.96 |
| Phosphoric acid (neutral) | 6.99 | 7.97 | 8.56 | | |

(continued)

| pH Adjusting agent | pH Value | | | | |
|---|---|---|---|---|---|
| Glycine-NaOH | 8.60 | 8.98 | 10.51 | | |
| Triethylamine | 9.73 | 10.53 | 11.63 | | |
| Phosphoric acid (alkaline) | 11.33 | 12.09 | 12.85 | | |
| NaOH | 13.90 | | | | |

(3) Measurement of platinum-gold colloidal particle

The amount of platinum-gold colloidal particles which were contained in the solution collected by the above operation was measured. Specifically, the collected solution was introduced into a sample cell, and scattered light obtained by irradiating the sample cell with a laser beam of 650 nm was measured , by a photodiode set at a predetermined position.

The intensity of the scattered light increases in proportion to the concentration of the platinum-gold colloidal particles in the solution, and accordingly, by measuring the intensity of scattered light, it is possible to quantitatively analyze the concentration of the platinum-gold colloidal particles in the solution. In other words, by treating the immunocomplex with a pH adjusting agent, the "moiety including platinum-gold colloidal particle" which was separated from the solid phase or the immunocomplex can be detected.

Note that in the case where the platinum-gold colloid-labeled anti-NP antibody non-specifically adsorbs (to a place other than the antigen) and is not removed by the washing treatment, the antibody is separated by the pH adjusting agent, and results in being mixed into the measurement sample. For this reason, only 100 $\mu$L of the platinum-gold colloid-labeled anti-NP antibody solution was added to a well in which the antibody was subjected to solid-phasing treatment in the same way as described above, and the mixture was allowed to stand still at 37°C for 10 minutes, and subsequently, the well was washed with TBS-T; then 100 $\mu$L of a pH adjusting agent solution was added thereto, and the well was allowed to stand still at room temperature for 2 minutes; and then, the solution was collected from the well, and was subjected to measurement of the scattered light. The same operation was performed for each pH adjusting agent, and the obtained intensity of the scattered light was used as a blank value for each pH adjusting agent. Note that in a case where the pH adjusting agent has weak capability of cleaving the complex, the difference between the intensity of the scattered light of the sample and the intensity of the scattered light of the blank becomes small, and accordingly, there is a case where the intensity of the scattered light of the measurement result of the sample is indicated by a negative value in the result of the difference between the two.

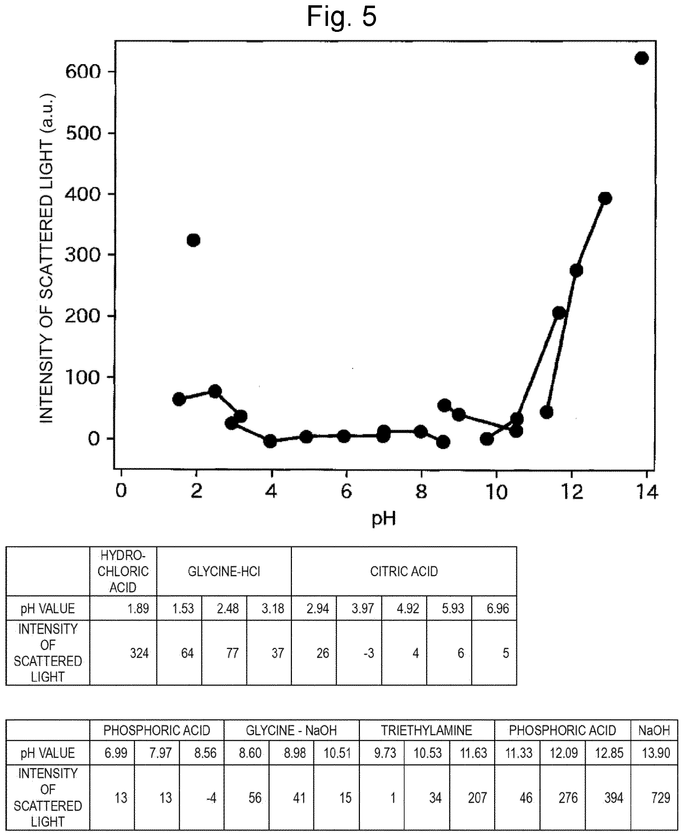

The blank value can be estimated as the intensity of the scattered light of the platinum-gold colloidal particle derived from the platinum-gold colloid-labeled anti-NP antibody which non-specifically adsorbed to the inside of the well, and accordingly, a value obtained by subtracting the blank value from the measured value was defined to be the intensity of the scattered light of the "moiety including the platinum-gold colloidal particles" of the formed immunocomplex. The results are shown in Figure 5.

Figure 5 is a graph showing a relationship between the pH value and the cleavage characteristic of each reagent. The lower part of Figure 5 shows the pH value of each reagent and the corresponding value of the intensity of the scattered light, and the upper part of Figure 5 shows each value by a graph. The horizontal axis of the graph is the pH value, and the vertical axis is the intensity of the scattered light. As can be seen from Figure 5, in the case where the pH value is in the vicinity of neutrality, the intensity of the scattered light is low. In other words, it is shown that the added neutral reagent is low in a cleaving performance for the "moiety including platinum-gold colloidal particle" of the complex. On the other hand, in the case where the added reagent is acidic or basic, the intensity of the scattered light increases; and it was shown that it was possible to cleave the "moiety including platinum-gold colloidal particle" of the complex (solid phase-anti-NP antibody-SARS-CoV-2NP-platinum-gold colloid-labeled anti-NP antibody) bound to the solid phase, by pH adjusting agents such as acids and bases.

In particular, it was shown that pH adjusting agents (acids) having a pH lower than pH 4 and pH adjusting agents (bases) having a pH of 10.6 or higher had satisfactory cleaving performances, and furthermore, pH adjusting agents (bases) having a pH of 11.6 or higher had excellent cleaving performances.

(Example 2) Separation of moiety including labeling substance (platinum-gold colloidal particle) by sodium hydroxide

As a result of Example 1, the cleaving capability of sodium hydroxide solution was particularly satisfactory, and accordingly, the performance of cleaving the moiety including the labeling substance from the complex (solid phase-anti-NP antibody-SARS-CoV-2NP-platinum-gold colloid-labeled anti-NP antibody) bound to the solid phase was further evaluated by changing the concentration of sodium hydroxide.

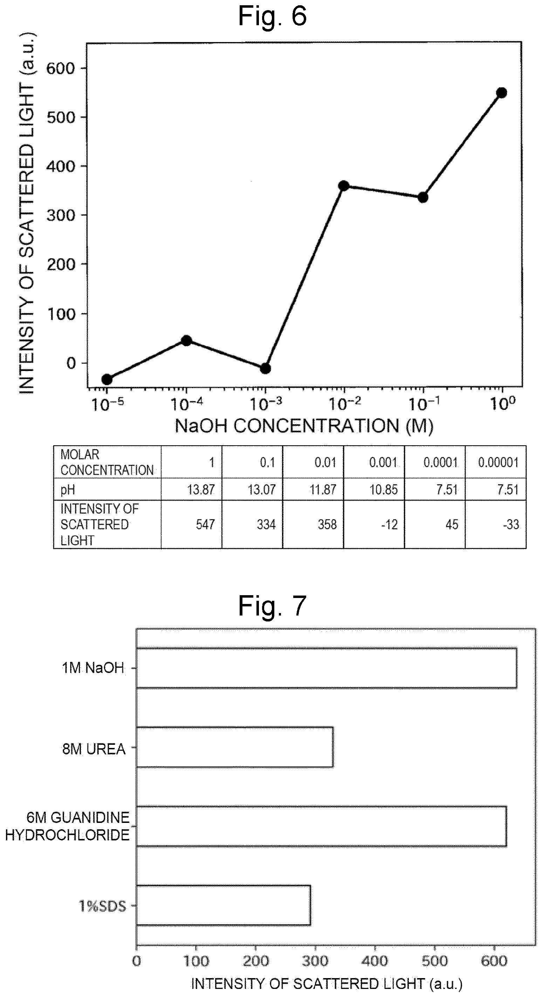

The sodium hydroxide concentrations were set at 0.01 mM, 0.1 mM, 1 mM, 10 mM, 100 mM, and 1 M. Then, the same treatment as in Example 1 was performed except that 100 μL of the aqueous solution of sodium hydroxide was used which was adjusted to each concentration. The results are shown in Figure 6.

Figure 6 is a graph showing the cleaving performance for the complex by sodium hydroxide. The lower part of Figure 6 shows a pH value and a value of the intensity of the scattered light at each concentration of sodium hydroxide, and the upper part of Figure 6 shows each value by a graph. The horizontal axis of the graph is a molar concentration of sodium hydroxide, and the vertical axis is the intensity of the scattered light.

As can be seen from Figure 6, it has become clear that sodium hydroxide separates (releases) the moiety including platinum-gold colloidal particle of the immunocomplex, at a concentration of 10 mM or higher. Note that a pH of a 10 mM sodium hydroxide solution was 11.87.

(Example 3) Separation of moiety including labeling substance (platinum-gold colloidal particle) by denaturing agent

A microplate was prepared that was provided with wells in which anti-NP antibodies were solid-phased and were subjected to blocking treatment, in the same method as in Example 1. Then, a solution in the well was collected in the same way as in Example 1 except that in place of the pH adjusting agent, a denaturing agent was allowed to act on the complex (solid phase-anti-NP antibody-SARS-CoV-2NP-platinum-gold colloid-labeled anti-NP antibody) which was formed on the wells of the microplates as solid phase, and the amount of platinum-gold colloidal particles was measured in the same way as in Example 1(3). Note that 8 M of urea, 6 M of guanidine-HCl, or 1% SDS was used as the denaturing agent. The results are shown in Figure 7. Note that the results of the same treatment which was performed with the use of 1M-NaOH are also shown in Figure 7, for comparison.

Figure 7 is a graph showing an action of the denaturing agent on the immunocomplex bound to the solid phase, by the intensity of the scattered light from the separated (released) platinum-gold colloid (moiety including platinum-gold colloidal particle). The case in which 1M-NaOH was used is shown as comparison data. As can be seen from Figure 7, it has become clear that any of the denaturing agents releases the moiety including the platinum-gold colloidal particle.

(Example 4) Sensitizing treatment of gold colloidal particle

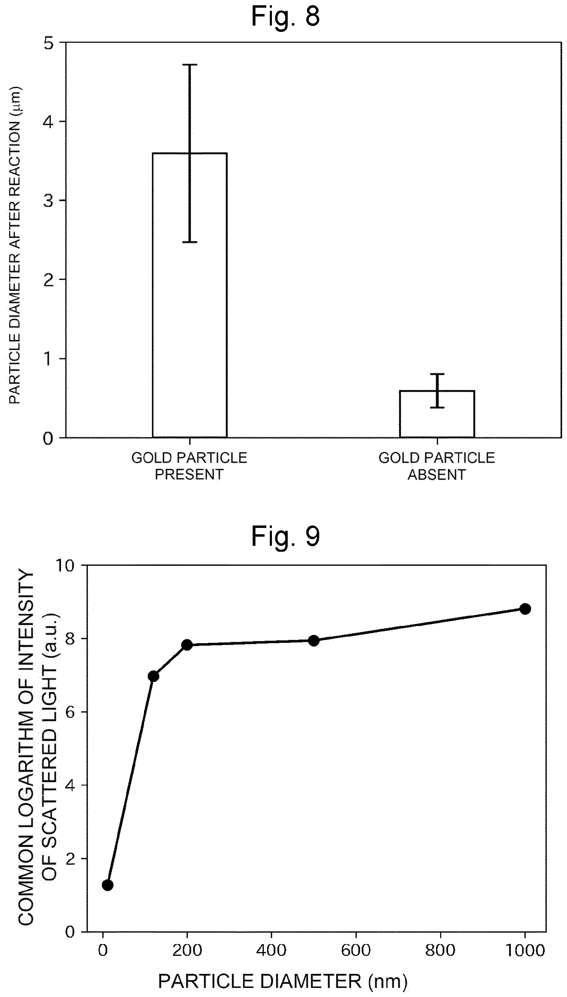

The gold colloidal particle suspension (particle diameter: about 12 nm) which was prepared in Reference Example 3 was adjusted to a concentration of 4.36 nM with pure water, and 2 μL thereof was added to 100 μL of 1M NaOH and mixed. This treatment by 1M NaOH was schematically performed as cleaving treatment with a pH adjusting agent for the complex. The above mixture was neutralized by addition of 100 μL of 1M HCl. This neutralized liquid in an amount of 6.35 μL was added to a mixed liquid of 972 μL of 10 mM of Walpole buffer (pH 2.0) and 1.65 μL of 12.1 M solution of chloroauric acid; 20 μL of 375 mM hydroxylamine hydrochloride was further added thereto; the mixture was mixed, and was allowed to stand still at room temperature for 10 minutes; and thereby, a sensitization reaction was performed. After that, the gold colloidal particles in the solution were sedimented by centrifugation, the supernatant was removed, and the precipitate was washed with ultrapure water. 1 μL of the dispersion liquid of thus obtained gold colloidal particles was dropped onto a sample holder on which a carbon tape was affixed, the air was removed, and the sample was photographed with an electron microscope TM4000Plus (manufactured by Hitachi, Ltd.). The applied voltage was set at 15 kv, and the magnification was set at 1000. Five or more visual fields were photographed, the images were analyzed, and thereby, the average particle diameter and the standard deviation were calculated. Note that the same sensitizing treatment as above was performed with the use of ultrapure water that did not contain gold colloidal particles, in place of the dispersion liquid of 4.36 nM gold colloidal particles, and the resultant was used as a control. The results are shown in Figure 8.

Figure 8 is a graph showing changes in diameters of gold colloidal particles due to the sensitization reaction. The vertical axis of Figure 8 shows the particle diameter. As can be seen from Figure 8, the sensitization reaction allowed the gold colloidal particles in the dispersion liquid of 4.36 nM gold colloidal particles to grow into particles having a particle diameter of about 3 to 5 μm. On the other hand, also in the case where the gold colloidal particles were not contained, the precipitation of gold colloidal particles was observed, but both were clearly distinguishable in the detection because the particles were remarkably small as compared with the case where the sensitizing treatment was performed. Note that the particle diameter can be naturally adjusted when the reaction conditions are adjusted, and accordingly, it is obvious that it is possible to produce gold colloidal particles having a particle diameter suitable for the detection system.

From this result, it has been shown that the diameter of the fine gold colloidal particle could be increased by the sensitization reaction, and furthermore that the gold (colloid) particle after the sensitization can be optically detected sufficiently.

(Example 5) Change in intensity of scattered light by particle diameter of metal particle

(1) Preparation of gold colloidal particles and platinum colloidal particles each having various particle diameters

Platinum-gold colloidal particles having a particle diameter of 120 nm were prepared according to Reference Example 1. In addition, gold colloidal particles having particle diameters of 12 nm and 200 nm were prepared according to Reference Example 3, and for gold (colloid) particles having particle diameters of 500 nm and 1000 nm, commercialized products (NANOPARTz, A11-500-CIT-DIH-1-50, and A11-1000-NPC-DIH-1-100) were acquired. The particles were each prepared in a state of being dispersed in a solution.

(2) Measurement of intensity of scattered light

The dispersion liquids of the gold colloidal particles having respective particle diameters, which were prepared in the above, were diluted to concentrations in a range in which scattered light could be measured, and the scattered light was measured by the same method as in Example 1. The intensity of the scattered light at the 1 nM was calculated by dividing an obtained value of the intensity of the scattered light by the concentration. As a result, it has been found that the intensity of scattered light increases according to an increase in the particle diameter of the gold colloidal particle. The results are shown in Figure 9.

Figure 9 is a graph showing a relationship between the particle diameter of the metal colloidal particle and the intensity of the scattered light. The horizontal axis shows the particle diameter (nm), and the vertical axis shows the intensity of the scattered light in logarithmic representation. As can be seen from Figure 9, the intensity of the scattered light remarkably increases from the 120 nm, and from the result, it has been shown that it is possible to detect the metal colloidal particles from about 100 nm by a simple scattered light detection system including a laser light source and a photodiode.

(Example 6) Detection of antigen based on moiety including labeling substance (platinum-gold colloidal particle)

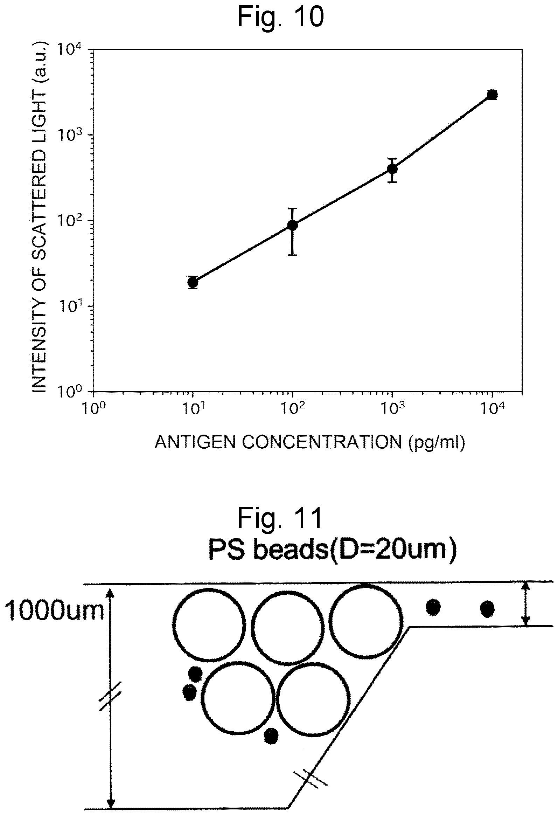

The anti-NP antibody which was used for the solid phasing in Example 1 was adjusted to 20 $\mu$g/mL with a tris-hydrochloric acid buffer solution, and an antibody dilution solution was produced. This solution was mixed with a magnetic particle (manufactured by JSR Life Sciences), and the anti-NP antibody was solid-phased on the magnetic particle by chemical bonding. Then, the anti-NP antibody solid-phased on the magnetic particle was allowed to react with each of SARS-CoV-2NP solutions which were adjusted to 10, 100, 1000 and 10000 pg/mL concentrations, and the platinum-gold colloid-labeled anti-NP antibody which was prepared in Example 1, at 37°C for 30 minutes. The magnetic particles were collected from each solution after the reaction, by centrifugal filtration using a 0.45 $\mu$m centrifugal filter (Merck), and were washed five times with TBS-T. After that, 1M-NaOH was added to the magnetic particle to perform the cleaving treatment (elution) of the complex (magnetic particle - anti-NP antibody - SARS-CoV-2NP - platinum-gold colloid-labeled anti-NP antibody) bound to the magnetic particle, and the intensity of the scattered light of the collected eluate was measured. The measurement results are shown in Figure 10. Note that in Examples, the term "elution" is not limited to the dissolution of separated part of the complex into a liquid, as long as a moiety including the labeling substance, which is a part of the complex, is cleaved from the complex, and as a result, the part is separated from the solid phase and released into the liquid.

Figure 10 is a graph showing a concentration of the eluted platinum-gold colloidal particle (moiety including platinum-gold colloidal particle) with the intensity of the scattered light. The vertical axis shows the intensity of the scattered light in logarithmic representation, and the horizontal axis shows an antigen concentration. As can be seen from Figure 10, the intensity of scattered light based on the platinum-gold colloidal particle was proportional to the antigen concentration. In other words, when the antigen concentration increased, also the amount of eluted platinum-gold colloidal particles increased; and accordingly, it was demonstrated that the cleaving treatment was effectively implemented on the complex formed on the solid phase by the antigen-antibody reaction, and further, the cleaved moiety including the labeling substance was separated, eluted, and appropriately detected.

(Example 7) Detection of scattered light emitted from rotating disk

(1) Experiment for evaluating outflow property of gold colloidal particle

In the present Example, an outflow property of the gold colloidal particle was experimentally evaluated. For this experiment, a disk was prepared that included a chamber which was partitioned on the center side of the circular plate, and a flow path which communicated with the chamber and extended outward in a radius direction of the circular plate. On this disk, a polypropylene sheet with a thickness of 50 $\mu$m (hereinafter abbreviated as "PP sheet") was in close contact with the upper surface of a circular plate in which a chamber and a flow path were formed in recessed forms, via an adhesive layer, and the upper surfaces of the chamber and the flow path were formed with the PP sheet.

Figure 11 is a schematic view showing a cross section of part of a flow path portion of the disk which was used in the present Example. The inside of the chamber was formed so that a depth in the thickness direction became 1000 $\mu$m, and the flow path communicated with the upper portion of the wall of the chamber on the opposite side of the rotation center. The flow path was formed so that a width was 230 $\mu$m and a depth was 15 $\mu$m, had a rectangular cross section of the flow path, and was formed with a capillary burst valve which opens to allow a liquid to flow therethrough when the disk rotates at a speed exceeding a predetermined number of rotations. In addition, as shown in Figure 11, a wall of the chamber in contact with the flow path was inclined so as to rise obliquely from the bottom surface. Because of this, the depth of the chamber gradually became shallow toward the flow path. In the prepared disk, a through hole which penetrated between the inside and outside of the chamber was drilled by puncturing the PP sheet on the upper surface of the chamber.

A dispersion liquid of polystyrene beads (PS beads) (manufactured by Micromod, 25 mg/ml water base dispersion liquid having particle diameter of 20 $\mu$m) was injected into the chamber through the drilled through hole. The amount of the injected dispersion liquid was small compared to the capacity of the chamber, and accordingly, a liquid pool was formed in the vicinity of the through hole by surface tension. Next, the disk was rotated at 2000 rpm for 1 minute, and a solvent in the chamber was discharged into the flow path. As for the solvent which passed through the flow path, the total amount was collected by being sucked with a pipette through a hole which was opened in the PP sheet on the upper surface of the chamber at the end of the flow path.

After that, 10 $\mu$L of the dispersion liquid of the gold colloidal particles prepared in Reference Example 3 was injected into the chamber through the through hole. Thereby, a liquid pool of the dispersion liquid of the gold colloidal particles was formed in the vicinity of the through hole by surface tension. Subsequently, the disk was rotated at 2000 rpm for 1 minute, the solution in the chamber was discharged into the flow path, and the liquid which passed through the flow path was collected from the chamber at the end of the flow path, in the same way. The collected liquids of the dispersion liquid of the gold colloidal particles, which was prepared in Reference Example 3, and the dispersion liquid of the PS beads were subjected to measurement of absorbance or intensity of the scattered light with a nano spectrophotometer or a measuring instrument for intensity of scattered light, respectively, and the two were compared. As a result, the PS bead was not contained in any of the collected liquid, whereas the collected liquid derived from the dispersion liquid of the gold colloidal particles contained gold colloidal particles exceeding 60% of the initial concentration. Thereby, it has been confirmed that the gold colloidal particles pass through the blocked PS beads and flow out from the chamber to the flow path.

(2) Specific detection of labeling substance with scattered light

Next, the light scattered by the labeling substance was detected in the case where a biomaterial coexisted. BSA was used schematically as a foreign substance. As the gold colloidal particles, the dispersion liquid was used that was produced in the same way as in Reference Example 3 and in which gold colloidal particles having a particle diameter of 200 nm were dispersed. A 10% solution of BSA (manufactured by Oriental Yeast Co., Ltd., Cat 47408903) or water was prepared as a solvent, and a dispersion liquid of 2 pM gold colloidal particles was diluted in each of the solution and water into 2 fM. The diluted dispersion liquid of the gold colloidal particles and the 10% BSA solution were each added to a 300 $\mu$L of cuvette, and the intensity of the scattered light was measured with the detection device for the scattered light, which was used in Example 1.

For this experiment, a disk that included a chamber which was partitioned on the center side of the rotation, and a flow path which communicated with the chamber and extended outward in a radius direction of the circular plate, was prepared. In this disk, a chamber and a flow path were formed in recessed forms in a circular plate made from resin, and a PP sheet having a thickness of 50 $\mu$m was in close contract with the upper surface of the circular plate via an adhesive layer. The upper surfaces of the chamber and the flow path were formed by the PP sheet. The chamber and the flow path were both formed so that the depth was 15 $\mu$m, and the bottom surfaces were continuous with each other. A width of the flow path was 1 mm, the leading end of the extended flow path was connected to a branch path orthogonal to the flow path, and the branch path was connected to a thin flow path heading toward the rotation center side, at both ends thereof. The thin flow path was connected to and communicated with the end portion of the chamber on the rotation center side, at the end portion of the chamber on the rotation center side. The chamber was formed wider than the flow path. In addition, a through hole was drilled in the upper surface of the chamber.

The BSA solution or the dispersion liquid of the gold colloidal particles which were diluted into 2fM with the BSA solution were injected into the chambers of the disks, respectively. The disk was set on the mounting table of the apparatus shown in Figure 4, and rotated at 2000 rpm for 2 minutes, and the number of scattered light occurrences was counted. The results are shown in Table 2.

Table 2

| Sample | Intensity of scattered light (a.u.) | Count |
|--------|-------------------------------------|-------|
| GNP | 67 | - |
| BSA | 121 | 0 |
| GNP+BSA | 217 | 104 |
| When corrected to water = 0 | | |

Table 2 shows the measurement results of the dispersion liquid of the gold colloidal particles ("GNP"), the BSA solution ("BSA"), and the dispersion liquid of the gold colloidal particles diluted with the BSA solution ("GNP + BSA") in order from the upper stage, and the middle column shows the measurement results of the intensity of the scattered light by the detection device of the scattered light, and the rightmost column shows the count results of the number of occurrences of scattered light emitted from the rotated disk. As can be seen from this result, in the measurement of the intensity of the scattered light with the use of the cuvette, the scattered light was measured in each of the dispersion liquid of the gold colloidal particles and the BSA solution, and stronger scattered light was measured in a sample in which both were mixed. From this result, it has been shown that, in such a measurement method in which a cell having a relatively large capacity such as a 300 μL cuvette is used and irradiated in a state of standing still with irradiation light to detect scattered light, it is difficult to specifically detect a detection target such as the gold colloidal particle by scattered light, in the case where a foreign substance exists. On the other hand, in the case where the disk was used, the measurement which used water as a blank and the measurement which used the BSA solution gave the same results, and the generation of scattered light due to the BSA was not counted (detected). Furthermore, in the case where the dispersion liquid of the gold colloidal particles was measured, the generation of scattered light derived from the gold colloidal particle was counted, and the detection of the gold colloidal particle was shown.

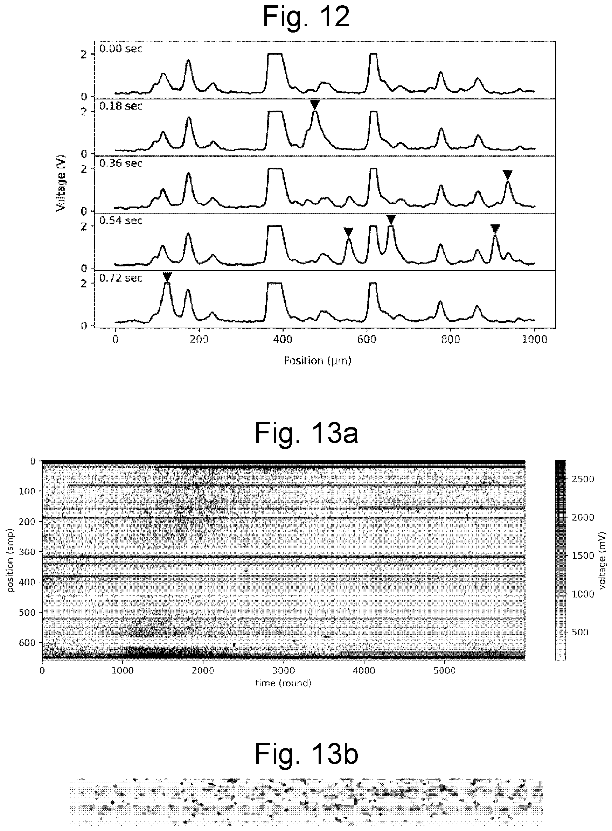

Figure 12 is a graph showing the results of detection of gold colloidal particles using the disk. The horizontal axis of Figure 12 shows the position (position) in a width direction of the flow path in the tubular channel, and the vertical axis shows the intensity of the scattered light by a voltage value. In addition, the peak of the counted gold colloidal particles is shown by ▼. In Figure 12, changes in the detection peak with elapsed time are shown from top to bottom. According to the graph, it is understood that there are peaks which do not change with elapsed time and are continuously observed, and intermittent peaks which appear only for a short time. The peaks which do not change with elapsed time can be determined to be noise. Accordingly, it is possible to cancel the noise by obtaining the intensity (voltage value) of the scattered light of the blank at the same position of the tubular channel, and calculating the difference between the intensity of the scattered light of the blank and the intensity (voltage value) of the scattered light obtained in an arbitrary round at the time of measurement. On the other hand, the intermittent peaks that intermittently occur can be determined to be based on a dynamic gold colloidal particle passing through the flow path. Therefore, it has been shown that even when a foreign substance exists, it is possible to separate and specifically detect the detection target of interest.

(Example 8) Separation and detection of scattered light of moiety including labeling substance (gold colloidal particle) with the use of rotating chip-type device

(1) Preparation of gold colloid-labeled antibody solution

A gold colloid-labeled anti-NP antibody was prepared in the same procedure as in the production of the platinum-gold colloid-labeled antibody in Example 1, except that the gold colloidal particle having a particle diameter of 200 nm which was prepared in Reference Example 3, was used as the labeling substance, in place of the platinum-gold colloidal particle. The produced gold colloid-labeled anti-NP antibody was suspended in a specimen extract liquid, and a gold colloid-labeled antibody solution was obtained.

(2) Preparation of polystyrene bead solid-phased antibody

Another antibody different from the anti-NP antibody that was used in the previously described gold colloid-labeled anti-NP antibody was selected, among the anti-NP antibodies which were prepared in Reference Example 2, and was bound to polystyrene beads (manufactured by Micromod, particle diameter of 20 μm, 25 mg/ml, and dispersed in water system) by a maleimide method, and a polystyrene bead solid-phased anti-NP antibody was prepared.

(3) Formation of immunocomplex having sandwiched form of antibody-antigen-antibody

SARS-CoV-2NP was used as an antigen, and the antigen, the above gold colloid-labeled antibody solution, and the above polystyrene bead solid-phased anti-NP antibody were each adjusted with the specimen extract liquid to predetermined concentrations. Then, these resultants were mixed and reacted at room temperature for 10 minutes, and immunocomplexes in a sandwiched form of antibody-antigen-antibody were formed.

(4) Washing treatment

A chip type device was prepared that includes, a reaction chamber, a flow path, a tubular channel, a drain reservoir, a washing liquid reservoir, and a reagent reservoir (cleaving reagent reservoir) similar to those in Figure 2b, and in which one unit was independently formed.
Then, the solution containing the immunocomplex prepared in the above was introduced into the chip type device. Specifically, the solution containing the immunocomplex prepared in the above was injected into the reaction chamber; the chip type device was set on the mounting table of the apparatus shown in Figure 4, and was rotated at 2000 rpm for 10 seconds; thereby the liquid was discharged from the reaction chamber; then, the rotation was stopped; and the washing liquid was injected into the reaction chamber. After that, the chip type device was rotated at 2000 rpm for 9 minutes, and the washing treatment was performed. After the washing treatment, the tubular channel was filled with the liquid. In addition, the washing liquid was filled and stored beyond the inlet of the tubular channel, to a position at which the polystyrene beads in the reaction chamber were almost immersed.

(5) Separation of moiety including labeling substance (gold colloidal particle) and specific detection of labeling substance by scattered light

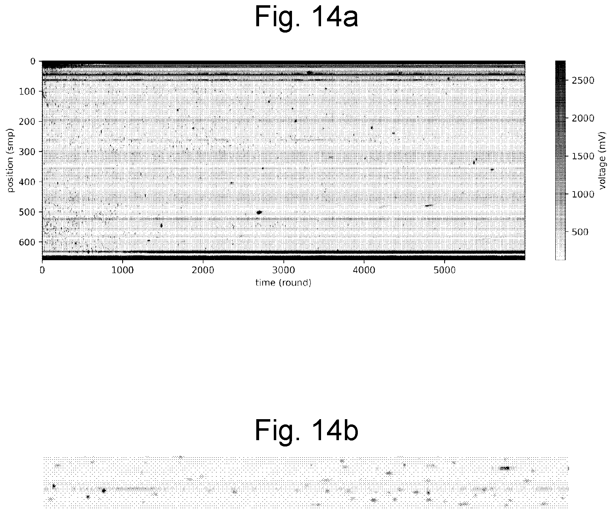

After the washing treatment, 1M NaOH was injected into the reaction chamber as a cleaving reagent. Then, the chip type device was rotated at 2000 rpm for 3 minutes, and thereby, the gold colloidal particle (moiety including gold colloidal particle) was separated from the complex, in other words, was eluted into a liquid which was stored in the reaction chamber. The gold colloidal particle which has been eluted into the liquid in the reaction chamber passes through the tubular channel by the centrifugal force, and at this time, generates scattered light by being irradiated with irradiation light; and the scattered light is detected. The detected scattered light is converted into an electric signal and acquired as a voltage value. Figure 13a and Figure 13b shows the detection results of scattered light in the case where the concentration of the antibodies was adjusted to 0.5 pg/mL and the immunocomplexes were formed.
Figure 13a and Figure 13b are views showing the results of detection for gold colloidal particles (moieties including gold colloidal particles) by scattered light, in the present Example. In Figure 13a, the horizontal axis shows a rotation time period (time (round)) of the chip type device, and 1000 time corresponds to 30 seconds. The vertical axis shows a position in a width direction of the tubular channel (position (smp: sampling)), and 100 smp corresponds to 492 $\mu$m. The voltage value corresponding to the detected scattered light is output and displayed as grayscale image data. The gradation of the gray scale corresponds to a level of the voltage value, and the brightness decreases as the voltage value increases. Figure 13b is a partially enlarged view of Figure 13a, and the horizontal axis shows a range of 1000 to 2000 round, and the vertical axis shows a range of 200 to 300 smp.
Here, a mixture was prepared as a sample, in which an antigen was not added, and polystyrene bead solid-phased anti-NP antibody was mixed with a gold colloid-labeled antibody solution, and was subjected to the above procedures of (4) washing treatment and (5) separation (elution) of the moiety including the labeling substance (gold colloidal particle) and specific detection of the labeling substance by scattered light, in the present Example; and the result was shown in Figure 14a and Figure 14b. The vertical axis, the horizontal axis, and the display data in Figure 14a and Figure 14b are the same as those in Figure 13a and Figure 13b, respectively, and Figure 14b is a partially enlarged view of Figure 14a.
The gold colloid-labeled anti-NP antibody introduced into the reaction chamber adsorbs (non-specifically adsorb) to a substance other than the antigen, in some cases. When NaOH (cleaving reagent) is added to the gold colloid-labeled anti-NP antibody that has non-specifically adsorbed to the substance, the moiety including the gold colloidal particle results in being separated from the adherend. This phenomenon occurs due to the addition of the cleaving reagent, and accordingly, a gold colloidal particle that is not a detection target is also introduced into the tubular channel together with a gold colloidal particle that is a detection target (a gold colloidal particle that has formed complex), and results in being unintentionally detected. However, it is possible to correct the obtained result, by previously grasping the amount of the gold colloid-labeled anti-NP antibodies which have non-specifically adsorbed to the substance by the method as described above.
Note that in Figure 13a and Figure 14a, a continuous strong signal which does not change with elapsed time is observed near both ends of the tubular channel in the width direction. The strong signal is recognized also in the Figure 14a, and accordingly, it is difficult to assume that the signal is due to the gold colloidal particle, and it can be determined that the signal is noise caused by refraction, scattering, distortion or the like of light, which occurs in the vicinity of the end portion of the tubular channel. In the case where such a signal is measured, the vicinities of both ends of the tubular channel are excluded from an effective range of detection.

In addition, the signal continuously detected at the same position (In Figure 13a and Figure 14a, the signal is displayed as a linear image because the signal is output with the same intensity along the time axis.) is the scattered light originating from something that is not moving through the tubular channel, and accordingly can be determined to be noise based on a flaw or the like in the tubular channel.

Furthermore, only the gold colloidal particles were allowed to flow through the tubular channel of the chip type device, the scattered light was detected, and the shapes of signals indicating the scattered light of the gold colloidal particles were confirmed from the obtained image data; and as a result, it was found that approximately circular minute spots correspond to the signals of the gold colloidal particles in Figure 13a to Figure 14b. From this result, it can be determined that an anisotropic shape or a slightly large dot-like spot observed in the Figure 14a is scattered light (noise) based on a foreign substance, because the shape is different from that of the signal derived from the gold colloidal particle. Therefore, such a noise signal can be excluded from the detection results.

On the other hand, it can be determined that the approximately circular minute spot that is intermittently generated is based on the scattered light derived from the dynamic gold colloidal particle which passes through the tubular channel. In the effective detection range excluding the vicinities of both ends in the width direction of the tubular channel, the spots corresponding to the gold colloidal particles were counted in each of the Figure 13a and Figure 14a.

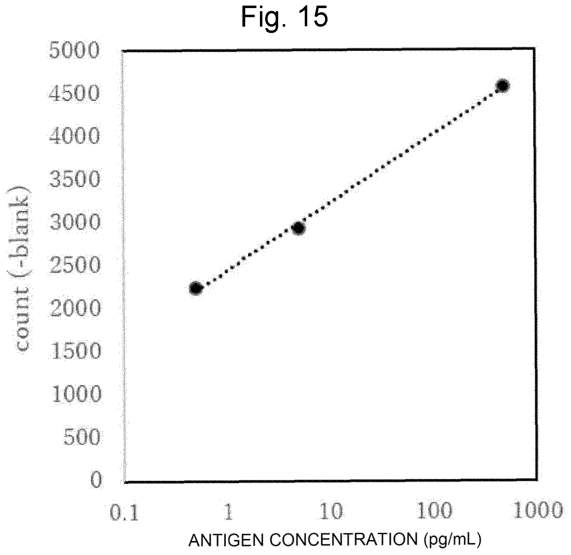

Then, the count number of the Figure 14a was subtracted from the count number of the Figure 13a, and the difference was determined to be the count number of the gold colloidal particles corresponding to the antigens. Figure 15 shows the relationship between the count number (count (- blank)) and the antigen concentration at the time when the immunocomplex was formed. The horizontal axis shows the antigen concentration in common logarithm, and the vertical axis shows the count number of the scattered light corresponding to the antigen.

As can be seen from the comparison between the Figure 13a and the Figure 14a, the number of spots that indicate the gold colloidal particles displayed in the Figure 14a is much more than that in the Figure 13a. Furthermore, as shown in Figure 15, the number of detected counts (count (- blank)) is a "positive value" of the order of a thousand, and shows clear significant difference as compared to the case where the antigen does not exist. Therefore, it has been shown that it is possible to separate the moiety including the labeling substance from the complex, by using the device, and implementing the washing treatment and the cleaving treatment with a cleaving reagent, for the immunocomplex fixed to the solid phase, and to detect the labeling substance by the scattered light which is derived from the moving labeling substance that is the separated moiety including the labeling substance, which has been moved in the tubular channel by the centrifugal force.

Furthermore, as shown in Figure 13b and Figure 14b, the spots corresponding to the gold colloidal particles can be clearly confirmed. In other words, it has been shown that it is possible to count the signal based on the scattered light of each gold colloidal particle, and to realize quantitative evaluation of the gold colloidal particles.

In particular, from Figure 15, in the case where the antigen concentration is low, such a tendency has been shown that the count number of scattered light based on the gold colloidal particles shows a satisfactory correlation with the antigen concentration. From this result, such a possibility has been shown as to be capable of more quantitatively detecting the gold colloidal particle corresponding to the antigen concentration, particularly in the case where the concentration of the antigen contained in the sample is low.

In order to investigate whether this method was able to detect antigens at lower concentrations as compared with conventional immunochromatography, the above results were compared with the results obtained by the conventional immunochromatography. ImmunoAce SARS-CoV-2 II (manufactured by Tauns Laboratories, Inc.) was used for the conventional immunochromatography, and the results of antigen detection were visually evaluated. Note that SARS-CoV-2NP was used as an antigen, of which the concentration was adjusted so as to become a predetermined value. The results are shown in Table 3.

Table 3

| Antigen concentration ($\mu$g/mL) | Immunochromatography | This detection method |
|---|---|---|
| 500 | + | + |
| 50 | + | + |
| 5 | ND | + |
| 0.5 | ND | + |
| 0.05 | ND | ND |

(continued)

| Antigen concentration (μg/mL) | Immunochromatography | This detection method |
|---|---|---|
| 0 | ND | ND |

In Table 3, the leftmost column shows the final concentration of the antigen which has been used when the immunocomplex has been formed. The middle column shows the results by the conventional immunochromatography. The rightmost column shows the results by the method in Example 8. The symbol "+" indicates that an antigen was detected in the immunochromatography, and indicates that scattered light was counted in the method of the present Example. "ND" indicates that the antigen was not detected by the immunochromatography, and that the scattered light was not counted by the method of the present Example. The lowest concentration of antigens for which the detection was confirmed was 0.5 pg/mL in the method of the present Example, but in contrast to this, the lowest concentration of antigens was 50 pg/mL in the conventional immunochromatography. From this result, it can be said that the method of the present Example can detect the antibody at a lower concentration and is excellent in sensitivity as compared with the conventional immunochromatography.

Note that in the method of the present Example described above, the washing liquid was directly injected into the reaction chamber from the outside at the time of the washing treatment, but in place of this method, it is also acceptable to store the washing liquid in the washing liquid reservoir, rotate the chip type device, and thereby supply the washing liquid from the washing liquid reservoir to the reaction chamber. In this regard, in an experiment implemented separately, the washing liquid was injected into the washing liquid reservoir, and the chip type device was rotated at 2000 rpm for 1 minute, and then it was visually confirmed that the amount of the washing liquid in the washing liquid reservoir decreased and the amount of the washing liquid in the reaction chamber increased. From this result, it has been shown that the washing liquid stored in the washing liquid reservoir is introduced into the reaction chamber by rotation, and can perform the washing treatment.

In addition, in the chip type device, a reagent reservoir (cleaving reagent reservoir) is arranged on the rotation center side with respect to the reaction chamber, and is connected to the reaction chamber by a flow path. The reagent reservoir is connected to the reaction chamber and the tubular channel by the similar flow path as in the washing liquid reservoir, and accordingly, from the above experiment of transferring the washing liquid by rotation, it can be said that it is possible to supply the cleaving reagent to the reaction chamber by the centrifugal force generated by rotation of the chip type device, and implement the cleaving treatment in the reaction chamber as in the case of the washing liquid, if also the cleaving reagent is stored in the reagent reservoir.

Industrial Applicability

The method according to the present invention can detect a target substance with high sensitivity and at low cost, and accordingly, can be used as a method and an apparatus for detecting a substance in various industrial fields in addition to a medical field.

Reference Signs List

1 disk
2 unit
10 reaction chamber
11 tubular channel
12 drain reservoir
13 washing liquid reservoir
14 cleaving reagent reservoir

**Claims**

1. A target substance detection method, comprising:

    forming a complex by sandwiching a target substance between a first trapping substance bound to a labeling substance and a second trapping substance immobilized on a solid phase, and separating a moiety including the labeling substance from the formed complex;

moving the separated moiety including the labeling substance through a liquid filled in a tubular channel by a centrifugal force; and

detecting the moiety including the labeling substance by light scattered by the moiety including the labeling substance from light irradiated into the tubular channel.

2. The target substance detection method according to claim 1, comprising:
implementing a washing treatment of washing the complex in a state in which the complex is prevented from flowing into the tubular channel, before the moiety including the labeling substance is separated from the complex.

3. The target substance detection method according to claim 2, wherein
when the washing treatment is implemented, the tubular channel is filled with a washing liquid used for the washing.

4. The target substance detection method according to claim 2, wherein
a washing liquid used for the washing treatment accumulates downstream from the tubular channel.

5. The target substance detection method according to claim 2, wherein
the complex is retained in a predetermined chamber, whereby the complex is prevented from flowing into the tubular channel.

6. The target substance detection method according to claim 5, comprising:

arranging a first reservoir that stores the washing liquid, the chamber, and a second reservoir that stores a liquid flowing out from the chamber, in a device formed to be rotatable, in this order from a rotation center side of the device toward an outer side; communicating the first reservoir and the chamber with each other through a self-opening and closing flow path, and communicating the chamber and the second reservoir with each other through the tubular channel;

rotating the device, thereby supplying the washing liquid from the first reservoir to the chamber and then discharging the washing liquid to the second reservoir via the tubular channel so as to implement the washing treatment.

7. The target substance detection method according to claim 6, wherein
the first reservoir and the chamber are communicated with each other through an openable flow path.

8. The target substance detection method according to claim 6, wherein
the washing treatment is performed until the second reservoir and the tubular channel are filled with the washing liquid.

9. The target substance detection method according to claim 8, comprising:

arranging a third reservoir that stores a liquid containing a reagent for separating the moiety including the labeling substance from the complex, in a side closer to the center of rotation than the chamber of the device while the third reservoir and the chamber communicate with each other through a self-opening and closing flow path; and rotating the device, thereby supplying a liquid containing the reagent to the chamber after the washing treatment, whereby the moiety including the labeling substance is separated and then the separated moiety including the labeling substance is moved into the tubular channel.

10. The target substance detection method according to claim 9, wherein
the third reservoir and the chamber are communicated with each other through an openable flow path.

11. The target substance detection method according to any one of claims 1 to 10, comprising:
detecting scattered light that is intermittently generated from the tubular channel within a predetermined time period.

12. The target substance detection method according to any one of claims 1 to 10, wherein
the labeling substance is a substance having a specific gravity larger than that of the liquid filled in the tubular channel.

13. The target substance detection method according to claim 12, wherein
the labeling substance is a fine particle that contains one selected from the group consisting of a metal, a ceramic, glass and a resin, as a main constituent component.

14. The target substance detection method according to claim 13, wherein
the labeling substance is a metal colloidal particle.

15. The target substance detection method according to any one of claims 1 to 10, wherein
the solid phase is at least one granular body selected from the group consisting of glass particles, ceramic particles, magnetic particles and resin particles.

16. The target substance detection method according to any one of claims 1 to 10, wherein
the solid phase is a structure of an inside of a container that is used for forming the complex.

17. The target substance detection method according to any one of claims 1 to 10, wherein
the moiety including the labeling substance is separated from the complex, by at least one treatment selected from the group consisting of heat treatment, pH adjustment treatment, denaturation treatment, oxidation treatment, reduction treatment, enzyme treatment and competitive reaction treatment.

18. The target substance detection method according to any one of claims 1 to 10, wherein
the first trapping substance is at least one selected from the group consisting of an antibody, an antibody fragment, a modified antibody, an antigen, an aptamer and a nucleic acid, each of which specifically binds to the target substance.

19. The target substance detection method according to any one of claims 1 to 10, wherein
the second trapping substance is at least one selected from the group consisting of an antibody, an antibody fragment, a modified antibody, an antigen, an aptamer and a nucleic acid, each of which specifically binds to the target substance.

20. A reagent for use in the target substance detection method according to claim 1, the reagent having an action of separating the moiety including the labeling substance from the complex.

21. The reagent according to claim 20, comprising:
at least one selected from the group consisting of a pH adjusting agent, a denaturing agent, a reducing agent, an oxidizing agent, an enzyme and a competing agent.

22. The reagent according to claim 21, comprising the pH adjusting agent.

Fig. 1

# Fig. 2a

( a )

# Fig. 2b

( b )

# Fig. 3

(a)

# Fig. 4

# Fig. 5

| | HYDRO-CHLORIC ACID | GLYCINE-HCl | | | CITRIC ACID | | | | |
|---|---|---|---|---|---|---|---|---|---|
| pH VALUE | 1.89 | 1.53 | 2.48 | 3.18 | 2.94 | 3.97 | 4.92 | 5.93 | 6.96 |
| INTENSITY OF SCATTERED LIGHT | 324 | 64 | 77 | 37 | 26 | -3 | 4 | 6 | 5 |

| | PHOSPHORIC ACID | | | GLYCINE - NaOH | | | TRIETHYLAMINE | | | PHOSPHORIC ACID | | | NaOH |
|---|---|---|---|---|---|---|---|---|---|---|---|---|---|
| pH VALUE | 6.99 | 7.97 | 8.56 | 8.60 | 8.98 | 10.51 | 9.73 | 10.53 | 11.63 | 11.33 | 12.09 | 12.85 | 13.90 |
| INTENSITY OF SCATTERED LIGHT | 13 | 13 | -4 | 56 | 41 | 15 | 1 | 34 | 207 | 46 | 276 | 394 | 729 |

## Fig. 6

| MOLAR CONCENTRATION | 1 | 0.1 | 0.01 | 0.001 | 0.0001 | 0.00001 |
|---|---|---|---|---|---|---|
| pH | 13.87 | 13.07 | 11.87 | 10.85 | 7.51 | 7.51 |
| INTENSITY OF SCATTERED LIGHT | 547 | 334 | 358 | -12 | 45 | -33 |

## Fig. 7

## Fig. 8

## Fig. 9

## Fig. 10

## Fig. 11
## PS beads(D=20um)

## Fig. 12

## Fig. 13a

## Fig. 13b

# Fig. 14a

# Fig. 14b

Fig. 15

## INTERNATIONAL SEARCH REPORT

| | International application No. |
|---|---|
| | **PCT/JP2023/016016** |

### A. CLASSIFICATION OF SUBJECT MATTER

***G01N 33/483***(2006.01)i; ***G01N 33/543***(2006.01)i; ***G01N 33/545***(2006.01)i; ***G01N 33/552***(2006.01)i; ***G01N 35/00***(2006.01)i; ***G01N 37/00***(2006.01)i

FI: G01N33/483 C; G01N33/543 501A; G01N33/543 541A; G01N33/543 541Z; G01N33/545 A; G01N33/552; G01N35/00 D; G01N37/00 101

According to International Patent Classification (IPC) or to both national classification and IPC

### B. FIELDS SEARCHED

Minimum documentation searched (classification system followed by classification symbols)

G01N33/483; G01N33/543; G01N33/545; G01N33/552; G01N35/00; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2012-527621 A (ABBOTT LABS.) 08 November 2012 (2012-11-08) <br> see entire text, all drawings, particularly, claims, paragraphs [0018]-[0072], fig. 1-8, etc. | 1-22 |
| A | JP 2021-113708 A (PHC HOLDINGS CORP.) 05 August 2021 (2021-08-05) <br> see entire text, all drawings, particularly, claims, etc. | 1-22 |
| A | JP 2012-255772 A (JVC KENWOOD CORP.) 27 December 2012 (2012-12-27) <br> see entire text, all drawings, etc. | 1-22 |
| A | JP 2006-505766 A (BURSTEIN TECHNOLOGIES, INC.) 16 February 2006 (2006-02-16) <br> see entire text, all drawings, etc. | 1-22 |
| A | JP 2017-518503 A (SPINCHIP DIAGNOSTICS AS) 06 July 2017 (2017-07-06) <br> see entire text, all drawings, etc. | 1-22 |

☐ Further documents are listed in the continuation of Box C.  ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 July 2023** | **18 July 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2023/016016**

| Patent document cited in search report | | | Publication date (day/month/year) | Patent family member(s) | | | Publication date (day/month/year) |
|---|---|---|---|---|---|---|---|
| JP | 2012-527621 | A | 08 November 2012 | US | 2010/0297778 | A1 | |
| | | | | see entire text, all drawings, particularly, claims, paragraphs [0043]-[0094], fig. 1-8, etc. | | | |
| | | | | WO | 2010/135330 | A1 | |
| | | | | EP | 2433128 | A1 | |
| JP | 2021-113708 | A | 05 August 2021 | US | 2023/0047389 | A1 | |
| | | | | see entire text, all drawings, particularly, claims, etc. | | | |
| | | | | WO | 2021/145388 | A1 | |
| JP | 2012-255772 | A | 27 December 2012 | US | 2012/0288408 | A1 | |
| | | | | see entire text, all drawings, etc. | | | |
| JP | 2006-505766 | A | 16 February 2006 | US | 2002/0151043 | A1 | |
| | | | | see entire text, all drawings, etc. | | | |
| | | | | WO | 2003/087827 | A2 | |
| | | | | EP | 1493014 | A2 | |
| JP | 2017-518503 | A | 06 July 2017 | US | 2017/0138823 | A1 | |
| | | | | see entire text, all drawings, etc. | | | |
| | | | | US | 2017/0138938 | A1 | |
| | | | | US | 2017/0138939 | A1 | |
| | | | | WO | 2015/193474 | A1 | |
| | | | | WO | 2015/193471 | A1 | |
| | | | | WO | 2015/193478 | A1 | |
| | | | | EP | 3158332 | A1 | |
| | | | | EP | 3158333 | A1 | |
| | | | | EP | 3158334 | A1 | |
| | | | | JP | 2017-518502 | A | |
| | | | | JP | 2017-518504 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06505802 A
- JP 2008185423 A
- JP 2002503331 A
- JP 2012255772 A
- JP PATENTLAIDOPENKOHYONO A